# EUROPEAN PATENT SPECIFICATION

(11) **EP 0 850 244 B1**
(45) Date of publication and mention of the grant of the patent: **31.07.2002**
(21) Application number: 96931804.7
(22) Date of filing: 12.09.1996
(51) Int. Cl.: C07H 3/06, C07K 14/45, C12N 5/20, A61K 31/70, C07K 16/20, G01N 33/569, C12P 21/08, A61K 39/002

(54) **TOXOPLASMA GONDII GLYCOCONJUGATES**
TOXOPLASMA GONDII GLYKOKONJUGATE
GLYCOCONJUGUES DE TOXOPLASMA GONDII

(30) Priority: 15.09.1995 EP 95202500
(43) Date of publication of application: 01.07.1998
(73) Proprietor: Organon Teknika B.V., 5281 RM Boxtel (NL)
(72) Inventor: KOOLEN, Marcus, Josephus, Marie, NL-3992 BZ Houten (NL); DAMM, Jan, Bas, Louis, NL-5345 RA Oss (NL); STRIEPEN, Boris, Philadelphia, PA 19104 (US); SCHWARZ, Ralph, T., D-35435 Wettenberg (DE)
(74) Representative: Hermans, F.G.M.
(86) International application number: EP9604034
(87) International publication number: WO9710249

(56) References cited:
- NATO ASI SERIES, vol. H78, 1993, pages 109-121, XP002021746 R.T.SCHWARZ ET AL.: "Recent Advances in the Glycobiology of Toxoplasma Gondii." cited in the application
- JOURNAL OF BIOLOGICAL CHEMISTRY, vol. 267, no. 17, 15 May 1992, MD US, pages 11721-11728, XP002021747 S.TOMAVO ET AL.: "A Family of Glycolipids from Toxoplams Gondii." cited in the application
- JOURNAL OF BIOLOGICAL CHEMISTRY, vol. 267, no. 30, 25 October 1992, MD US, pages 21446-21458, XP002021748 S.TOMAVO ET AL.: "Biosynthesis of Glycolipid Precursors for Glycosylphosphatidylinositol Membrane Anchors in a Toxoplasma Gondii Cell-free System."
- BIOCHEMICAL SOCIETY TRANSACTIONS, vol. 20, no. 3, August 1992, page 296s XP002021749 B.STRIEPEN ET AL.: "Identification and Characterisation of Glycosyl-Inositolphospholipids in Toxoplasma Gondii."
- BIOLOGY OF THE CELL, vol. 78, no. 3, 1993, pages 155-162, XP002021750 S.TOMAVO ET AL.: "Structural Analysis of Glycosyl-Phosphatidylinositol Membrane Anchor of the Toxoplasma Gondii Tachyzoite Surface Glycoprotein gp23."
- RESEARCH IN IMMUNOLOGY, vol. 144, no. 1, 1993, pages 24-31, XP002021751 R.T.SCHWARZ ET AL.: "The Current Status of the Glycobiology of Toxoplasma Gondii : Glycosylphosphatidylinositols, N- and O-linked Glycans."
- BIOCHEMICAL JOURNAL, vol. 291, no. 3, 1 May 1993, pages 713-721, XP002021752 M.ODENTHAL-SCHNITTLER ET AL.: "Evidence for N-Linked Glycosylation in Toxoplasma Gondii."

## Description

The present invention relates to a glycoconjugate immunoreactive with *Toxoplasma gondii* (to be referred to as *T. gondii* hereinafter) antibodies, (monoclonal) antibodies against these glycoconjugates, and cell lines capable of producing monoclonal antibodies. The invention is further concerned with immunochemical reagents and methods for the detection of *T. gondii* or antibodies directed against *T. gondii*. Furthermore, the invention relates to a therapeutic reagent for the inhibition of replication of *T. gondii* parasites and a method for the production of a live attenuated *T*. *gondii* vaccine.

### Introduction:

*Toxoplasma gondii* is an intracellular protozoan parasite found throughout the world and capable of infecting all species of mammals and all types within a given individual. *T. gondii* is classified as a coccidian with two life cycles, asexual and sexual.

In the asexual stage, *T. gondii* exists in different forms, for instance; tachyzoites, pseudocysts, bradyzoites, and oocysts. The tachyzoite is the obligate intracellular form of *T. gondii* which characterizes acute infection. The infective stage of *T*. *gondii* for human host cells is the pseudocyst. The pseudocyst has a diameter of 30-100 micrometers and contains hundreds to thousands of infectious units termed bradyzoites. Infection is frequently initiated by ingestion of pseudocysts present in raw or uncooked unfrozen meats. In addition, infection can occur by ingestion of oocysts in the faeces of cats experiencing active intestinal infection. Infections may also be acquired through blood or leukocyte transfusion, by organ transplantation or by transplacental transmission during pregnancy (Wilson *et al*., J. Exp. Med. 151, 328-346, 1980). The wall of either the pseudocyst or the oocyst is broken down in the small intestine by host digestive enzymes releasing the bradyzoites or sporozoites, respectively, which then penetrate the columnar epithelium. It is probable that bradyzoites or sporozoites reach the liver by the hematogenous route where they are ingested by Küpffer cells. Once inside a cell, the organisms are referred to as tachyzoites. Liver parenchymal cells also become infected (Kranenbühl & Remington, Immun. of Parasitic Infections; eds. Cohen, S., Warren, K.S. London: Blackwell Scientific Publications pp. 356-421, 1982; Remington & Kranenbühl, Immun. of Human Infection, part II, Edited by Nahmias, A.J., O'Reilly, R.J. New York, Plenum Medical Book Company, pp. 327-371, 1982).

Replication immediately follows entry into host cells. In the host, macrophages transport bradyzoites throughout the body. The bradyzoites survive and replicate within the macrophage parasitophorous vacuole by preventing the fusion of lysosomes with it. Replication results in the lysis of the host cell. Organisms are phagocytosed by new macrophages or other cell types and repeat the cycle.

The sexual stage of *T. gondii* occurs only in feline hosts (Miller *et al*., *J.* Parasitology, 58, p. 928-937, 1972). The intermediate host (e.g., mouse) becomes infected by ingesting either oocysts or pseudocysts. The mouse develops pseudocysts throughout its own tissues. The cat becomes infected when it eats infected meat (e.g., rodent tissues) containing the pseudocysts or ingests oocysts. Bradyzoites or sporozoites penetrate columnar epithelial cells and differentiate into merozoites. Following replication, merozoites rupture infected epithelial cells and infect adjacent ones. Some merozoites differentiate into pre-sex cells termed macrogametocytes (female form) and microgametocytes (male form). The microgametocytes fuse with macrogametocytes, forming zygotes termed oocysts (Dubey & Frenkel, J. Protozool. 19:155-177, 1972). Oocysts enter the lumen of the small intestine and are defecated. Each oocyst sporulates in the soil, producing eight infectious sporozoites, the infectious stage for the intermediate host.

The bradyzoite is the form that encysts approximately 8-10 days after acquisition *in vivo* and characterizes the chronic, latent phase of infection. Thus, the bradyzoite form is the only stage that has the ability to initiate the enteroepithelial cycle (Krahenbühl & Remington, 1982; Remington & Krahenbühl, 1982).

*T. gondii* induces a mild or unapparent disease in healthy adults but causes a severe disease, toxoplasmosis, or even death in congenitally infected children and in immunocompromised patients. Primary infection of pregnant women occurs in European countries with frequencies between 0.2 and 1.0%. In approximately 40-50% of the cases, the unborn child is infected. Infection in the fetus during pregnancy will (in approximately 10% of the cases) lead to neonatal death or a severely multi-handicapped child, but in 90% of the cases the child will be born with an asymptomatic, latent infection (Desmonts and Couvreur, Ann. Pediatr. 1984, 31, 805-809; Alford *et al*. Bull. NY Acad. Med. 1974, 50, 160-181). Up to 85% of the patients with latent congenital toxoplasmosis will develop significant sequelae including one or more episodes of active retinochoroiditis. Other clinical symptoms are inflammation, lymphadenitis, encephalitis and fever.

Congenital acquired toxoplasmosis can be diagnosed by demonstrating the presence of neonatal anti-*T*. *gondii* IgM and/or IgA antibodies or *T. gondii* specific nucleotides by nucleic acid amplification techniques (Burg *et al*., J. Clinical Microbiol., 27, 1787-1792, 1989; Savva *et al*., J. Med. Microbiol., 32, 25-31, 1990).

There are several strains of *T. gondii* known. One of the most important strains is the RH-strain which is highly virulent and originally isolated from human brain tissue.

Most investigations in the past regarding *T. gondii* were focused on the identification and molecular characterization of tachyzoite-specific antigens. More than 1000 different *T. gondii*-specific proteins have been identified. Membrane-anchored surface proteins and secreted/excreted proteins of *T. gondii* have been studied extensively since these antigens are highly immunogenic. Among the surface proteins, p30 is most abundantly present and constitutes approximately 5% of the total tachyzoite protein of the *T*. *gondii* RH strain. The p30 protein is recognized intensively by human IgM, IgG, IgA and IgE antibodies (Decoster *et al*., Clin. Exp. Immunol., 73, 376-382, 1988; Godard *et al*., Infect. Immun., 58, 2446-2450, 1990) and is therefore useful for diagnostic purposes. Using monoclonal antibodies directed against p30, two immunocapture tests have been developed for the detection of anti-Toxoplasma IgM (Cesbron *et al*., J. Immunol. Methods, 83, 151-158, 1985) and IgA (Decoster *et al*., Lancet, ii, 1104-1106, 1988) antibodies. Another immunodominant antigen of *T. gondii* having a molecular weight of 4-10 kDa, has been identified. The 4-10 kDa antigen is recognized predominantly by human IgM and to a lesser extent by human IgG antibodies (Sharma *et al*., J. Immunol. 131:977-983, 1983). Furthermore, it was observed that treatment of membrane components with NalO4, an agent known to oxidize vicinal hydroxyl groups on sugar moieties, considerably reduced the immunoreactivity of *T. gondii* antigens with human IgM as well as with IgG antibodies in an ELISA (Naot *et al*., Infect. Immun. 41, 331-338, 1983). Sharma *et al*. (J. Immunol. 131:977-983, 1983) observed that NaIO4-treated Toxoplasma sonicates or membrane-enriched fractions, separated on SDS gels, showed a dramatic reduction in immunoreactivity of the immunodominant *T. gondii* antigens with human IgM and IgG antibodies.

Especially, the immunoreactivity of the 4-10 kDa antigen with human anti-*T*. *gondii* antibodies was completely destroyed following NaIO4-treatment. Furthermore, the 4-10 kDa molecule is insensitive to pronase and trypsine treatment but sensitive to lipase indicating that it constitutes a glycolipid structure. In summary, the studies outlined above indicate that the carbohydrate molecules of *T*. *gondii* play a major role in inducing an efficacious immune response in the infected host.

### Glycobiology of glycosylphosphatidylinositol (GPI)

It is now widely recognized that posttranslational modifications of proteins by oligosaccharides and glycolipids play an important role in the structure, biosynthesis and biological function of surface membrane proteins of viruses, bacteria and eukaryotic cells, including parasitic protozoa.

Proteins can be anchored to membranes via a stretch of hydrophobic amino acids spanning the lipid bilayer (transmembrane) or through covalent coupling to a glycosyl-phosphatidylinositol (GPI) molecule during the translocation of the nascent protein into the lumen of the rough endoplasmatic reticulum. In all reported cases, the lipid anchor replaces a short sequence of mainly hydrophobic amino acids at the carboxyl terminus of the protein and hence should be thought of as an alternative anchoring mechanism to the transmembrane polypeptide domain of type-1 membrane proteins (Ferguson and Williams, Ann. Rev. Biochem. 57, 285-320, 1988; Ferguson, Curr. Opinion in Struct. Biol., 1, 522, 1991).

Most of the information on the structure and biosynthesis of glycolipid membrane anchors has been obtained from studies of the variant surface glycoproteins (VSG) and GPI of the parasitic protozoan, *Trypanosoma brucei*. The glycolipid anchor of one VSG (VSG 117) has been completely chemically defined and consists of the backbone sequence ethanolamine-phosphate-6Manα1-2Manα1-6Manα1-4GlcNα1-6-inositol. The inositol residue is linked via a phosphodiester bond to dimyristoylglycerol, the ethanolamine group is amide-linked to the carboxyl terminal amino acid of the mature protein and a galactose side-chain is attached to the mannose residue adjacent to glucosamine. Similar backbone structures with different side-chain modifications and lipophilic groups have been described for the lipid anchors of some other proteins (for reviews, see Ferguson and Williams, Ann. Rev. Biochem. 57: 285-320, 1988; Cross, Ann. Rev. Cell Biol. 6: 1-39, 1990; Ferguson, Curr. Opinion in Structural Biology 1, 522-529, 1991).

### The glycosylphosphatidylinositol anchors of T.gondii

Until recently, very little on the glycobiology of *Toxoplasma gondii* was known and considerable controversy as to the presence of glycoproteins as major surface components of the Toxoplasma has existed. Handman *et al*. (J. Immunol. 124, 2578-2583, 1980) failed to observe any differences in the composition of membrane antigens after passage of detergent lysates of radioiodinated parasites over a ConA-sepharose column. In contrast, Johnson *et al*. (Biochem. Biophys. Res. Commun. 100, 934-943, 1981) and Mauras *et al*. (Biochem. Biophys. Res. Commun. 97, 906-912, 1980), using radiolabelled ConA, detected the binding of this lectin to Toxoplasma.

It is of fundamental importance to compare protein bound GPIs with their putative precursor molecules in order to establish a detailed biosynthetic pathway. The data presented by Schwarz *et al*. (NATO ASI Series. Series H: Cell Biology, vol. 78, Berlin, Springer, 1993. p. 109-121) suggest that p23 of *T. gondii* tachyzoites contain the highly conserved backbone, consisting of ethanolamine-PO4-6Manα1-2Manα1-6Manα1-4GlcNα1-6PI with a side-chain modification which was identified as a N-acetyl-galactosamine residue, similar to the mammalian Thy-1 anchor (cf Ferguson *et al*., Science 239: 753-759, 1988; Zinecker *et al*., Diplomarbeit, University of Marburg, 1992).

A comparable analysis was undertaken for p30 with analogous results (Tomavo *et al*., J. Biol. Chem. 267: 11721-11728, 1992). Thus the anchors of both proteins are identical with respect to their carbohydrate moieties. Some microheterogeneity for the glycan of P30 has been demonstrated (Zinecker *et al*., 1992) and it remains to be investigated if this is a common feature of toxoplasmal GPI-membrane anchors.

Since antibodies directed against GPI anchors of *T. gondii* do not bind to GPI anchors of other protozoa species it is anticipated that GPI anchors of *T. gondii* have unique constituents in addition to the more basic ethanolamine-P-6Manα1-2Manα1-6Manα1-4GlcNα1-6Inositol core structure.

Therefore, it is important to elucidate in detail the structure of the GPI-membrane anchors with respect to their glycan and lipid moieties and to search for structural features not found in mammalian cells.

When it became clear that the major surface proteins of *T. gondii* are anchored to the parasite surface by GPI-anchors (Tomavo *et al*., Mol. Cell. Biol. 9: 4576-4580, 1989; Nagel *et al*., J. Biol. Chem. 264: 5569-5574, 1989) the biological function and biosynthesis of these membrane anchors and, in particular, of their putative precursors became of major importance in this art. Moreover, the observation that the highly immunogenic 4-10 kDa antigens represent precursor species of uncomplexed or free glycosylphosphatidylinositol anchors boosted the interest in elucidating the biochemical structure and mapping the epitope(s) on GPI anchors of *T*. *gondii*. Information concerning the structure and immunodominant epitopes of GPI anchors of *T. gondii* will enable to chemically synthesize and apply artificial GPI anchors in diagnostic assays. In contrast to the native GPI anchors, artificial carbohydrate molecules resembling the immunoreactive sites can be chemically modified enabling direct coupling to enzyme conjugates by means of linker molecules.

Therefore, for the development of a specific and sensitive method to enable a reliable diagnosis to be made in various phases of the infection with *T. gondii* it is of great importance to identify immuno-dominant glycoconjugates responsible for the exact anchoring of specific membrane antigens.

By elucidating the structure of *T. gondii* GPI anchors it will be possible to interfere in the biosynthesis and linkage of the unique carbohydrate substitutes to the GPI core structure by inhibiting or blocking the function/activity of enzymes involved. This implies that specific inhibition or blocking of the GPI anchor synthesis of *T. gondii* will in consequence result in reduced cell surface expression of proteins and ultimately may be lethal for the parasite. For example the GPI precursor of *T. brucei* is remodelled with respect to its fatty acids. This remodelling is unique to the trypanosome and presents to be a good target for the development of rhemotherapeutics such as the myristate analogue 10-(propoxy) decanoic acid which shows selective toxicity to the trypanosome (Doering *et al*., Science 252, 1851-1854, 1991; Masterson *et al*., Cell 62, 73-80, 1990).

The role of GPIs in the biology and pathology of the parasite remains to be elucidated in detail. However, by elucidating the structure of the GPI-anchors, a new approach in the development of anti-Toxoplasma drugs based on interfering with parasite-specific carbohydrate structures may become possible.

The primary function of GPI anchors is to afford the stable association of proteins with the plasma membrane or, in a few cases, with the lumenal face of secretory granules. This feature of GPI anchors can be used to express heterologeous gene encoded proteins on the surface of *T*.*gondii*. Several studies have demonstrated that the efficiency of membrane targeting and turnover is solely determined by the GPI anchors. Therefore, by elucidating the structure of the GPI anchors of *T*. *gondii,* heterologeous genes can be modified in such a way that they will be efficiently linked to GPI anchors through which membrane targeting will be feasible. Ultimately, the applicability of the parasite *T*.*gondii* as a heterologeous gene expression system may lead to an efficient and reliable approach of vaccine development. Furthermore, GPI anchored proteins have a stable association with membranes, but they may sometimes be more readily detergent-solubilized than intrinsic membrane proteins (Hooper and Turner, Biochem. J., 250, 865-869, 1988). By using *T. gondii* as a heterologeous gene expression system, to express protein chimeras with GPI anchors, a reliable antigen source can been created which is easy to scale up and the expressed heterologeous proteins can be readily extracted with detergents (CHAPS, Triton X-114).

Results of experiments with B-cell deficient (IgM-suppressed) BALB/C mice have implied that anti-toxoplasma antibodies, although not essential for recovery in acute infection, may be necessary in controlling long-term toxoplasmosis (Frenkel and Taylor, Infection & Immunity, 38, 360-367, 1982). Data showed that glycolipid structures are T-helper cell independent B cell mitogens, which in consequence result in continuous IgM antibody producing B cells. Therefore, detailed information concerning the structure of GPI anchors of *T. gondii* will enable the synthesis of artificial GPI anchor molecules linked to appropriate ligands which can be applied as mitogens in evoking a protective antibody response in the susceptible host of *T*. *gondii*.

### Detailed description of the invention:

It has now been found, and in contrast with earlier publications in this art, that several glycoconjugates are immunoreactive with *Toxoplasma gondii* specific antibodies comprising a glycan core structure having the general formula 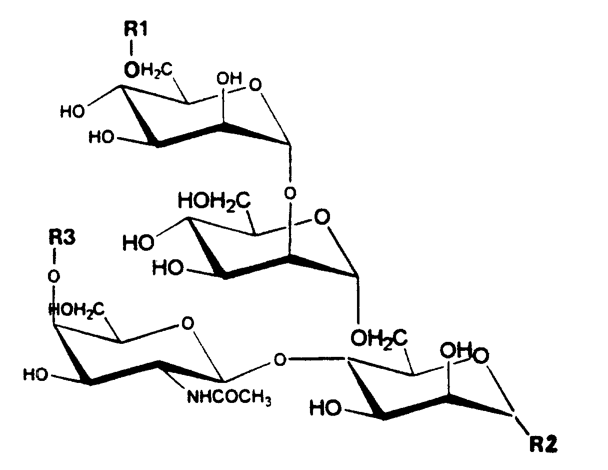 wherein R1= Hydrogen, -PO₃-CH₂-CH₂-NH₂ (ethanolamine-phosphate), or -PO₃-CH₂-CH₂-NH-X, wherein X= a *T. gondii* specific antigen;
wherein R2= α-O-R4, α(1-4)-O-anhydromannitol or α(1-4)-O-glucoseamine optionally α(1-6)-linked to the inositol moiety of a phosphatidylinositol membrane anchor;
wherein R3= a monosaccharide moiety,
wherein R4= Hydrogen or -L-R5, wherein L= a bivalent organic radical and
R5 = a functional group that allows coupling to a carrier.

The term 'bivalent organic radical' according to the present invention usually comprises a linker structure through which the functional group R₅ is attached to the glycan core. Suitable bivalent organic radicals are, for example, substituted or unsubstituted alkylene groups or aralkylene groups, such as methylene [-CH₂-], ethylene [-CH₂-CH₂₋] and, preferably propylene [-CH₂-CH₂-CH₂-].

The term 'functional group that allows coupling to a carrier' according to the present invention usually comprises any reactive functional group that can be used for covalent coupling of the glycan core structure of the invention to a carrier, or a functional group that can be converted into a reactive functional group for that purpose. Specific functional groups are mercapto (-SH), amino (-NH₂), pyridyldithio, S-acetyl-mercaptoacetyl (SATA), maleimido, a carboxylic acid function or an activated ester thereof, such as for example an ester derived from N-hydroxysuccinimide, p-nitrophenol or pentafluorophenol, bromoacetamido, vinylpyridine, hydrazide, aldehyde or hydroxy. Further suitable functional groups are know to those in the art of conjugation chemistry.

The term 'carrier' according to the present invention usually comprises any substance to which the glycan core structure of the invention can be conjugated, such as for example proteins (for instance, albumin or keyhole limpet hemocyanine) and solid supports that are used in diagnostic reagents.

A preferred embodiment of the present invention is a glycoconjugate wherein said monosaccharide moiety is a hexose moiety.

Another preferred embodiment of the present invention is a glycoconjugate wherein said hexose moiety is a glucose moiety.

Another preferred embodiment of the present invention is a glycoconjugate wherein said glucose moiety is bonded to the GalNAc-moiety by an α-1,4 glycosidic bond.

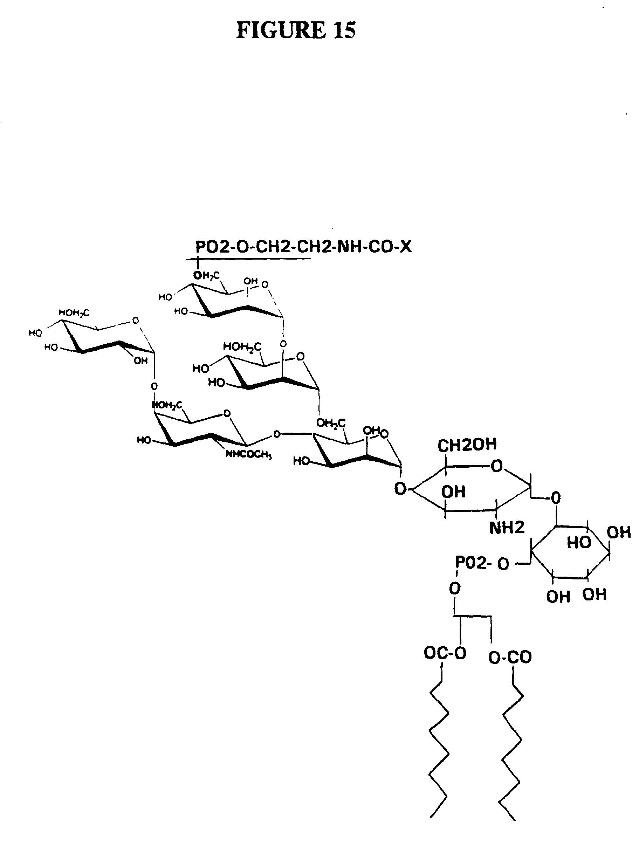

Another preferred embodiment of the present invention is a glycoconjugate wherein said glycoconjugate comprises the structure as shown in figure 15.

Another preferred embodiment of the present invention is a glycoconjugate obtainable from *T. gondii* tachyzoites by an isolation procedure comprising the steps of:
- disruption of the tachyzoite membrane, and,
- extracting the resulting tachyzoite suspension with a chloroform/methanol/water (C/M/W: 1/1/0.3 v/v) mixture.

Another preferred embodiment of the present invention is a glycoconjugate which is immunoreactive with human monoclonal antibodies produced by the immortalized cell-line deposited with the European Collection of Animal Cell Cultures (ECACC), Porton Down (UK), under deposit number 95090608 or 95090609.

Glycoconjugate is a generic term used to describe molecules in which carbohydrate moieties (mono-, oligo- or polysaccharides) are covalently attached to other (bio)molecules such as lipids or polypeptides. However, also carbohydrate core structures have to be considered to fall within the meaning of glycoconjugates.

Oligosaccharides linked to lipids (such as alkyl/acylglycerol, diacylglycerol, sphingosine, ceramide or dolichol) are known as glycolipids (IUB-IUPAC recommendations, 1976, (1976) J. Lipid Res. 19, 114). Glycoproteins are polypeptides to which oligosaccharides are bonded through N-and/or O-glycosidic linkages and in which the carbohydrates may represent from 2-60% of the overall weight (Montreuil *et al*., 1982, Comprehensive Biochemistry, vol. 19B, part II, p.1, ed. Florkin, G.). The oligosaccharide moieties present on these polypeptides are discrete, specific and conserved structures. This property distinguishes them from other glycoconjugates, such as proteoglycans, glycosaminoglycans, mucoproteins and peptidoglycans, where amino acids are linked to carbohydrate chains composed of variable numbers of repeating structural units (IUB-IUPAC recommendations, 1980, (1982) J. Biol. Chem. 257, 3352).

A further class of glycoconjugates are glycosylphosphatidylinositol (GPI) lipid anchors (Low *et al*., 1986, Trends Biochem. Sci. 11, 212). These link cell surface glycoproteins to membrane lipids through an oligosaccharide bridge. Thus, glycoproteins may be glycosylated with N- and O-linked oligosaccharides, as well as a GPI-anchor (Ferguson *et al*., 1988, Science 239, 753-759).

Glycoconjugates can be isolated and purified from a natural source (e.g. in the present invention from *T. gondii* parasites, and preferably from *T. gondii* tachyzoites). An isolation procedure as described in the examples, can be used. However, other isolation procedures can also be used which are known in the art and will therefore fall within the scope of the present invention.

Another strategy to obtain the glycoconjugates according to the present invention is a chemical synthetic route, using known methods frequently used in this art. In one of the examples is one synthetic route exemplified. However, other synthetic routes known in the art have to be considered equivalent and will therefore fall within the scope of the present invention.

Antibodies, directed to a glycoconjugate acccording to the invention are also part of the present invention.

The glycoconjugates prepared and described above can be used to produce antibodies, both polyclonal and monoclonal. Monoclonal antibodies directed against glycoconjugates according to the invention can be readily produced by one skilled in the art.

The monoclonal antibodies according to the present invention, therefore, provide a new means for the diagnosis of *T. gondii* infection.

Preferred antibodies according to the invention are monoclonal antibodies which bind to an epitope of the *T. gondii* glycoconjugate, which epitope is recognized by monoclonal antibody TOX.HuOT-1 or TOX-HuOT-2 produced by the hybridoma cell lines deposited with the European Collection of Animal Cell Cultures (ECACC), Porton Down (UK), under deposit No. 95090608 and 95090609 respectively.

Preferred antibodies according to the invention are monoclonal antibody TOX.HuOT-1 or TOX-HuOT-2 produced by the hybridoma cell lines deposited with the European Collection of Animal Cell Cultures (ECACC), Porton Down (UK), under deposit No. 95090608 and 95090609 respectively.

Immortalized cell lines capable of excreting monoclonal antibodies according to the invention are also part of the present invention.

The preparation of cell lines producing monoclonal antibodies may occur by, for example, by the Kohler and Milstein technique (Kohler and Milstein devised the techniques that resulted in the formation monoclonal antibody-producing hybridomas (G. Kohler and C. Milstein, 1975, Nature 256:495-497; 1976, Eur. J. Immunol. 6:511-519)), transformation with Epstein-Barr Virus, or a direct transformation technique of B-lymphocytes with oncogenic DNA, or a direct fusion of human B-lymphocytes with a fusion partner being either a human or a mouse-human hybrid myeloma cell line, or a direct fusion of an EBV-transformed B cell line with said myeloma cell lines.

Preferred cell lines according to the invention are the cell lines deposited at the European Collection of Animal Cell Cultures, Porton Down (UK) under deposit No. 95090608 and No. 95090609.

These hybridoma cell lines were produced with the aid of the Epstein Barr virus (EBV). EBV is capable of transforming and immortalizing human B-lymphocytes. Immortalized human B-lymphocytes can be obtained, with the aid of EBV, without the need of a myeloma partner cell. The Epstein Barr virus can be obtained from a variety of sources. The most common used source of EBV is the B95-8 marmoset cell line. The B95-8 cell line spontaneously releases EBV into the medium.

Monoclonal antibodies to glycoconjugates of *T. gondii* are useful tools for the detection of *T. gondii* expression in cells and cell extracts both in vivo and in vitro, for purification purposes and for a variety of biochemical and immunological analysis techniques to study the function of these proteins.

An immunochemical reagent comprising glycoconjugates or antibodies according to the invention is also part of the present invention.

The term "immunochemical reagent" according to the invention usually comprises one or more glycoconjugates according to the present invention and a suitable support or a labelling substance.

Supports which can be used are, for example, the inner wall of a microtestplate well or a cuvette, a tube or capillary, a membrane, filter, test strip or the surface of a particle such as, for example, a latex particle, an aldehyde particle (such as a ceramic magnetizable particle with active aldehyde surface groups), an erythrocyte, a dye sol, a metal sol or metal compound as sol particle, a carrier protein such as BSA or KLH.

Labeling substances which can be used are, inter alia, a radioactive isotope, a fluorescent compound, an enzyme, a dye sol, metal sol or metal compound as sol particle.

In a method for the detection of antibodies directed against *T. gondii* in a sample, an immunochemical reagent according to the invention is brought into contact with the sample. After which, the presence of immune complexes formed between the glycoconjugate and antibodies in the sample is detected and by this detection the presence of *T. gondii* antibodies in the sample is known and can be determined quantitatively.

Depending on the nature and further characteristics of the immunochemical reagent the immunochemical reaction that takes place is a so called sandwich reaction, an agglutination reaction, a competition reaction or an inhibition reaction.

For the detection of *T. gondii* in a sample an immunochemical reagent according to the invention, containing glycoconjugate according to the invention, can be brought into contact with the sample and anti-*T*. *gondii* antibodies after which the presence of immune complexes formed can be detected and, from this, the presence of *T*. *gondii* in a sample can be determined.

A particularly suitable method for the detection of *T. gondii* in a sample is based on a competition reaction between a glycoconjugate according to the invention provided with a labeling substance and an *T. gondii* antigen (present in the sample) whereby the glycoconjugate and the antigen are competing with the antibody directed against *T. gondii* attached to a solid support.

The invention further comprises a method for the detection of *T. gondii* in a sample characterized in that an antibody according to the invention is brought into contact with a sample whereafter the presence of immune complexes formed is detected which is a measure for the presence of *T. gondii* in the sample.

A test kit according to the invention comprises as an essential constituent an immunochemical reagent as described above. Carrying out a sandwich reaction, for the detection of *T. gondii* antibodies, the test kit may comprise, for example, the glycoconjuage according to the invention coated to a solid support, for example the inner wall of a microtestplate well, and either a labeled glycoconjugate according to the invention or a labeled anti-antibody.

For carrying out a competition reaction, the test kit may comprise a glycoconjugate according to the invention coated to a solid support, and a labeled antibody directed against *T*. *gondii* preferably a monoclonal antibody directed against said glycoconjugate.

In an agglutination reaction the test kit comprises an immunochemical reagent which may comprise a glycoconjugate according to the invention coated to particles or sols.

Another embodiment of a test kit is, for example, the use of a labeled glycoconjugate according to the invention as immunochemical reagent in a competition reaction with an *T. gondii* antigen to be detected for a binding site on the antibody directed against *T. gondii,* which is coated to a solid support.

The present invention further comprises a therapeutic reagent for the inhibition of replication of *T. gondii* parasites, wherein said therapeutic reagent comprises a specific inhibitor which inhibits the biosynthesis of the glycoconjugate according to our invention. An example of a specific inhibitor is a compound which is able to inhibit the enzyme which is specific and essential for the incorporation of the glucose moiety in the *T*. *gondii* glycoconjugate. This compound can be a chemical compound but also other compounds can be used which are known for their ability to inhibit specific enzymes. Inhibition of GPI-anchor (bio)synthesis can be achieved by means of genetic manipulation through which genes, encoding enzymes involved in the GPI-anchor synthesis, are eliminated (knock-out mutants). This can be achieved at the level of transcription or translation.

The present invention further comprises a method for the production of a live-attenuated *T. gondii* vaccine, wherein the surface expression of GPI-anchored antigens is blocked or inhibited by the therapeutic reagent according to the present, invention as explained above. Inhibition or blocked surface expression of GPI-anchored antigens will result in reduced virulence of *T. gondii.* Furthermore, so-called knock-out mutation of the GPI-anchor synthesis can be applied as live-attenuated vaccines.

Vaccines for the protection against *Toxoplasma gondii* are also part of the present invention. These vaccines comprise a glycoconjugate according to the invention, together with a pharmaceutical acceptable carrier comprising mitogenic properties.

In some cases the ability to raise protective immunity using these glycoconjugates per se may be low. In order to raise their immunogenicity, conjugation of these glycoconjugates to carrier molecules is then preferred. Suitable carriers for this purpose are macromolecules, such as natural polymers (proteins like key-hole limpet hemocyanin, albumin, toxins), synthetic polymers like polyamino acids (polylysine, polyalanine), or micells of amphiphilic compounds like saponins.

The vaccine according to the invention can be administered in a conventional active immunization scheme: single or repeated administration in a manner compatible with the dosage formulation and in such amount as will be prophylactically effective, i.e. the amount of immunizing antigen or recombinant micro-organism capable of expressing said antigen that will induce immunity against challenge by virulent *Toxoplasma gondii* parasites. Immunity is defined as the induction of a significant level of protection in a population after vaccination compared to an unvaccinated group.

The administration of the vaccine can be done, e.g. intradermally, subcutaneously, intramuscularly, intraperitoneally, intra-venously, orally or intranasally.

Additionally the vaccine may also contain an aqueous medium or a water containing suspension, often mixed with other constituents, e.g. in order to increase the activity and/or shelf life. These constituents may be salts, pH buffers, stabilizers (such as skimmed milk or casein hydrolysate), emulsifiers, adjuvants to improve the immune response (e.g. oils, muramyl dipeptide, aluminiumhydroxide, saponin, polyanions and amphipatic substances) and preservatives.

The invention is further exemplified by the following examples:

### Examples:

### Example 1

### Large scale preparation of Toxoplasma gondii tachyzoites

The RH strain of *Toxoplasma gondii* (to be referred to as *T*. *gondii* hereinafter), is the most commonly used laboratory strain *in Toxoplasma research.* Large scale preparation of *T. gondii* tachyzoites was carried out by infecting cell factories (6000 cm²) containing Vero cells with the tachyzoites at a multiplicity of infection of 0.30-1.0. After 3 days of culturing at 37°C, the medium was removed and cells refed with fresh medium. Six days after infection, culture supernatants were harvested and remaining Vero cells were washed with 200 ml phosphate-buffered saline solution (PBS). Supernatants were pooled and tachyzoites were filtered on 10-µm polycarbonate membranes (Nucleopore, Pleasanton, CA, USA) and washed twice with PBS. Tachyzoites were spun down (1,200 xg, 5 min.) and stored at -70°C.

Metabolic labeling of tachyzoites was performed essentially as described by Tomavo *et al*. (1992, JBC, 267, 11721-11728). Briefly, confluent monolayers of Vero cells (250 cm²) were infected with 5x10⁷ tachyzoites in Dulbecco's Modified Eagle Medium (DMEM) supplemented with 2% fetal calf serum. At 72 hours after infection, cell cultures were washed twice with glucose-free DMEM containing 20 mM sodium pyruvate. The labeling was performed by using the same medium supplemented with 0.5 mCi [³H]glucosamine for 4 hours at 37°C. After labeling, the parasites were liberated from host cells with 20 strokes in a Dounce homogenizer. The tachyzoites were purified by glass wool filtration as described (Grimwood *et al*., Exp. Parasitol. 48, 282-286, 1979).

### Example 2

### Immunoreactivity of glycoconjugates

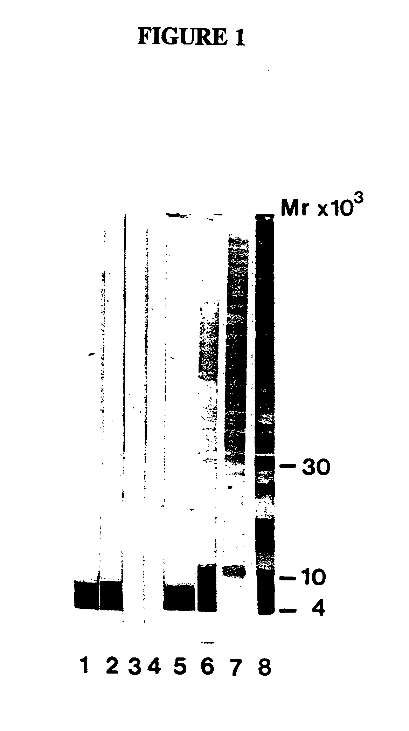

The immunoreactivity of glycoconjugates, and more specifically GPI molecules, with human anti-*Toxoplasma* IgM antibodies, was demonstrated by analyzing purified tachyzoites on SDS-PAGE (Sodium Dodecyl Sulphate-Polyacrylamide Gelelectrophoresis) and Western blots. Binding sites on the nitrocellulose filters were blocked by incubating the filters for 1 hr at ambient temperature with Phosphate-buffered Saline (PBS) solution supplemented with 20% fetal calf serum. Subsequently, filter strips were incubated with human anti-*Toxoplasma* IgM monoclonal antibodies directed against a 4-10 kDa fraction of T.gondii previously reported (Sharma *et al*., J. Immunol. 131, 977-983, 1983). Besides, a pool of five human anti-*Toxoplasma* IgM fractions obtained by affinity chromatography using Protein G Sepharose 4 Fast Flow (LKB Pharmacia, Uppsala, Sweden) as ligand, was evaluated as well. After an incubation period of 2 hrs at room temperature, strips were washed with Phosphate-buffered Saline Tween^{R} (PBST) and incubated for 1 hr with alkaline phosphatase conjugated goat anti-human IgM antibodies. Filter strips were washed and immunecomplexes were demonstrated by adding 5-bromo-4-chloro-3-indolyl phosphate and nitro blue tetrazolium (BCIP/NBT, Promega Corp., Madison, WI). Both human anti-Toxoplasma IgM monoclonal antibodies designated TOX.HuOT-1 and TOX.HuOT-2, recognized low molecular weight antigens represented by at least three bands (Figure 1, lane 1 and 2). Whereas the pooled IgM antibodies recognised a variety of *T*. *gondii* antigens, the human IgM monoclonal antibodies do not recognise GPI molecules linked to *T.gondii* proteins such as the immunodominant antigen p30. In addition to the monoclonal antibodies TOX.HuOT-1 and TOX.HuOT-2, two other monoclonal IgM antibody producing cell lines have been prepared from the same *T*. *gondii* infected patient. These IgM antibodies designated TOX.HuOT-3 and TOX.HuOT-8 were reactive with Toxoplasma antigens in the Toxonostika^{R} IgM ELISA (Organon Teknika N.V., Belgium) but are not reactive with the GPI molecules on the Western blot strips (Figure 1, lane 3 and 4).

Furthermore, two mouse monoclonal antibodies designated mAb T54E10 and mAb T33F12 are directed against the glycolipid fraction of T. gondii (Figure 1, lane 5 and 6). Both human and mouse monoclonal antibodies directed against glycolipid recognize solely the unlinked GPI molecules. None of the GPI-molecules linked to *T*. *gondii* proteins like for example p30 are recognized by the monoclonal antibodies tested. A mouse anti-human chorionic gonadotrophin hormone (lane 7) and a pool of five human anti-Toxoplasma IgM fractions (lane 8) were used as negative- and positive control respectively.

These data strongly suggest that both the human IgM epitope(s) as well as the mouse antibody epitope(s) on the GPI molecule is (are) no longer accessible via steric hindrance when GPI is covalently coupled in an amide bond to α-COOH group of the final amino acid of a protein, or is (are) destroyed following formation of the protein-ethanolamine bridge.

### Example 3

### Identification of GPI species

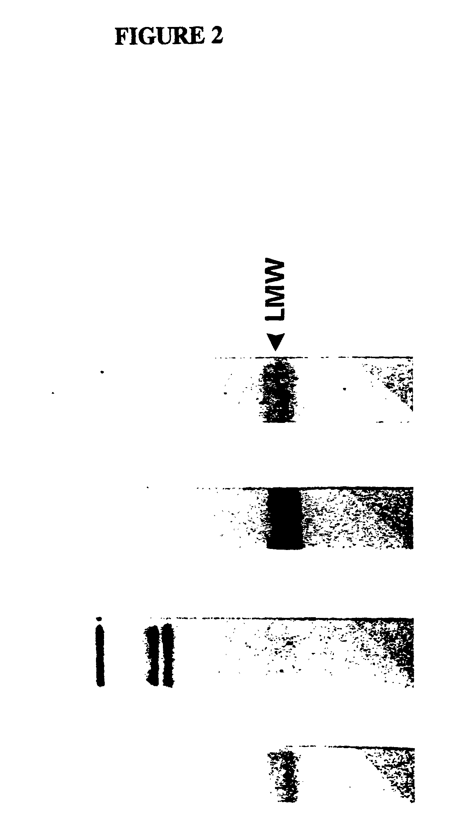

*T. gondii* tachyzoites were labeled as previously described (Tomavo *et al*., J. Biol. Chem., 267, 11721-11728, 1992; Tomavo *et al*., J. Biol. Chem., 267, 21446-21458, 1992) with ³H-glucosamine, ³H-mannose, ³H-palmitic acid, ³H-myristic acid, ³H-stearic acid and ³H-ethanolamine. Glycolipids were isolated according to extraction procedures previously published (Menon *et al*., EMBO. J., 9, 4249-4258, 1990; Tomavo *et al*., 1992). Briefly, tachyzoites were extracted twice with chloroform/methanol (CM; 2:1) to remove neutral lipids, phospholipids, and less polar glycolipids. The CM-residual pellet was then extracted twice with chloroform/methanol/water (CMW; 10:10:3) in order to obtain more polar lipids. The CMW-extracted glycolipids were pooled and purified by partitioning between water and water-saturated n-butanol. The immunoreactivity of the n-butanol fraction was analysed after SDS-PAGE and electrophoretical transfer on nitrocellulose membranes by using human IgM and IgG antibody containing serum sample as well as the two mouse monoclonal antibodies T33F12 and T54E10. This study revealed that human anti-*Toxoplasma* IgM antibodies did strongly recognize the GPI species whereas human IgG hardly did (Figure 2).

The glycolipids containing n-butanol phase, obtained from in vivo labeling described above, were spotted individually on prerun Silica 60 HPTLC plates (10x20 cm, Merck) and developed with the solvent system n-hexane/chloroform/methanol/water/acetic acid (3:10:10:2:1).

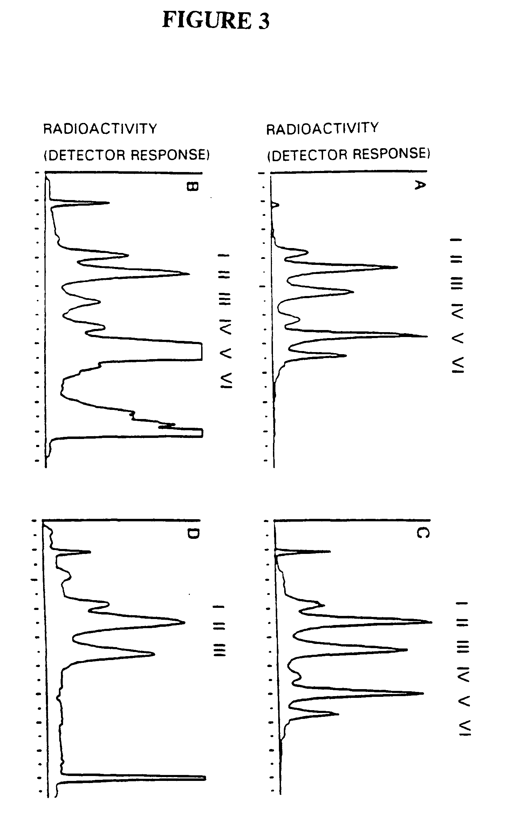

The chromatograms were dried and scanned for radioactivity using a Berthold LB 2842 linear analyser. By analysing n-butanol fractions of tachyzoites metabolically labeled with either ³H-glucosamine, ³H-mannose, ³H-stearic or ³H-palmitic acid, six different peaks were consistently detected on the TLC plates (Figure 3). However, only three peaks of radioactivity could be demonstrated when tachyzoites were labeled with ³H-ethanolamine (Figure 3D) indicating that three out of the six separated GPI species i.e. peak fraction I, II, III contained an ethanolamine-phosphate molecule.

Butanol phase containing glycolipids labeled with ³H-glucosamine was dried and taken up in 100 mM Tris/HCl pH 7.4 containing 0.1% sodium deoxycholate and 2 mM CaCl2 supplemented with 1 U PI-PLC (*Bacillus cereus*, Boehringer), 10 µl rabbit serum (as source of PLD), 50 U PLA2 (bee venom, Sigma) or 10 µl buffer respectively. Samples were incubated for 12 h at 37°C and boiled for 5 min to inactivate the enzymes. The reaction mixture was partitioned between water and butanol and the butanol phase was analysed by TLC. All glycolipids were sensitive to GPI-PLD and PI-PLC confirming their GPI structure. The sensitivity towards PLA2 indicates that the lipid moiety of the *T. gondii* glycolipids contains diacylglycerol.

### Example 4

### Immunoreactivity of the different GPI species

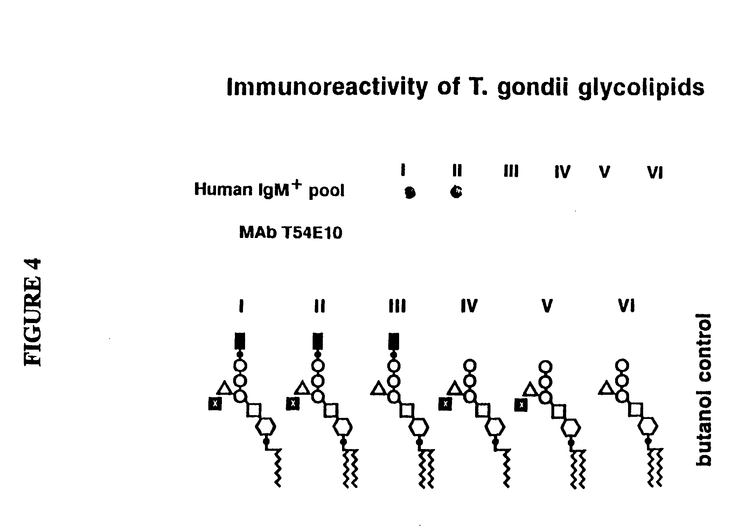

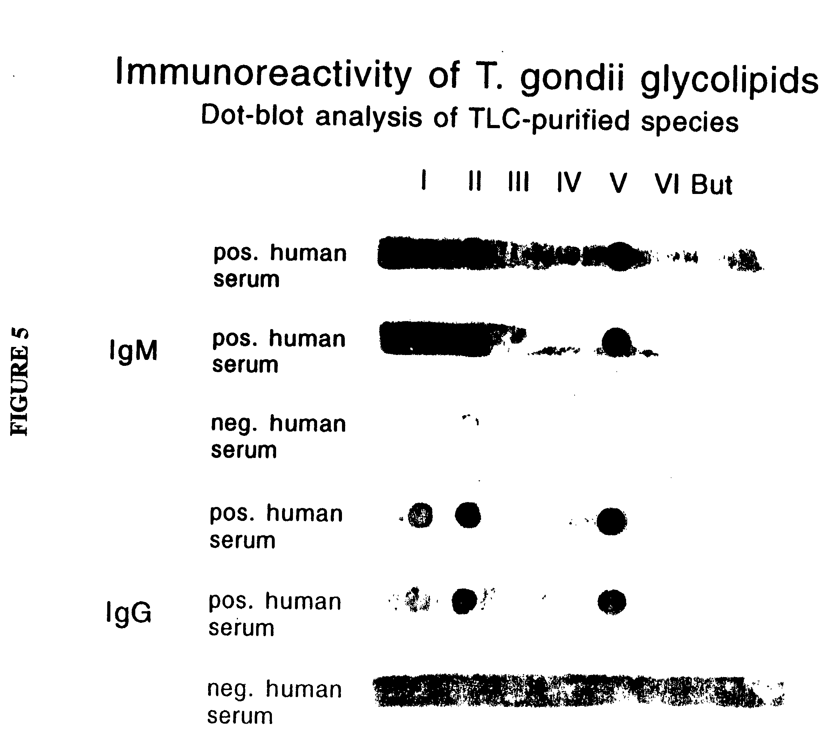

In order to show which peak fraction represents a GPI species recognized by human IgM antibodies indiviual glycolipid species were purified by preparative TLC using glycolipids metabolically labeled with ³H-glucosamine as a tracer. Areas of developed TLC-plates corresponding to the peaks of radioactivity were scraped, reextracted twice with 1.5 ml of CMW and partitioned between water and water saturated n-butanol in order to remove residual silica. The different GPI species (representing material derived from 2x10⁷ tachyzoites) were spotted onto reinforced nitrocellulose membranes (Scheicher and Schuell) in 10 µl butanol. Nitrocellulose strips were dried at 37°C and blocked overnight in 5% w/v non-fat dry milk in Tris buffered saline (15 mM Tris-HCl, pH 8, 140 mM NaCl, 0.05% w/v Tween 20). Blots were incubated with Toxoplasma patients sera as well as with mAb T54E10. After washing blots were incubated with an appropriate anti-Ig-alkaline phosphatase or anti-Ig-peroxidase conjugate and developed with BCIP and NBT for alkaline phosphatase (Promega) or using chemiluminescense detection for peroxidase (ECL, Amersham) using the suppliers protocols. As shown (Figure 4 and 5), human antibodies, and more specifically human IgM and IgG antibodies, reacted consistently with peak fractions I, II, IV, and V. The mAb T54E10 reacted with peak fractions I and II (Figure 4).

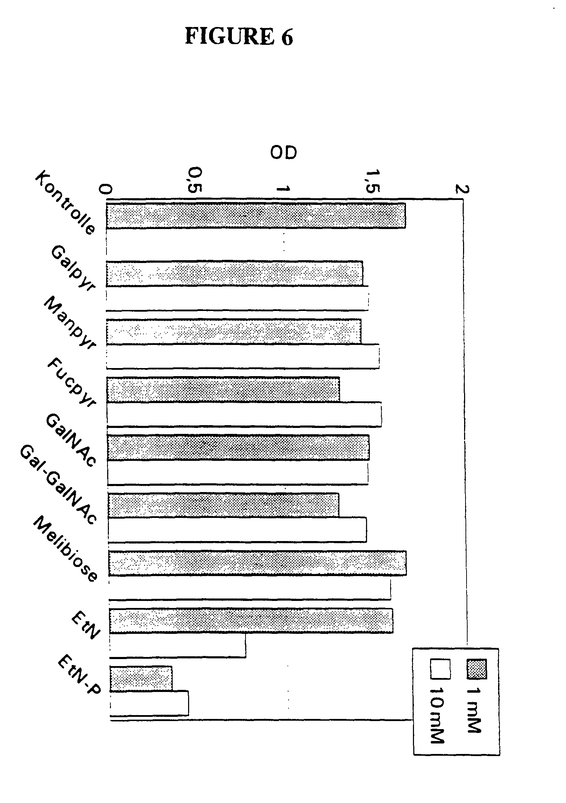

ELISA inhibition-binding studies were performed to demonstrate whether the free ethanolamine-phosphate group is part of the human IgM epitope on the GPI molecule. The GPI species of the n-butanol fraction were coated on microtiter wells at a coat concentration which corresponds to 10⁶ tachyzoites per well. As indicated in Figure 6, only the binding of mAb T54E10 to the n-butanol species was inhibited by ethanolamine-phosphate. Ethanolamine (10 mM) itself was able to inhibit the binding of mAb T54E10 by 50%. All other compounds tested were not able to inhibit the binding of the monoclonal antibody. However, the binding of human anti-Toxoplasma antibodies was not affected by any of the compounds tested.

### Example 5

### Analysis of the structure of the GPI species using metabolically labeled and TLC purified glycolipids

To generate hydrophilic headgroups, TLC purified glycolipids labeled with [³H]glucosamine were dried and resuspended in 400 µl freshly prepared 0.1 M sodium acetate (pH 3.5) containing 0.25 M NaNO₂ and were incubated at room temperature for 4 hours. The reaction was terminated by the addition of 300 µl of 0.4 M boric acid. Material deprived of the lipid moiety was recovered in the aqueous phase after butanol/water phase partition. This material was reduced using NaBH₄ and desalted over AG50W-X12 as previously described (Field & Menon, Lipid modification of proteins, Hooper & Turner, Oxford, 155-190, 1992). Headgroups were resuspended in 100 µl of 50 mM NaAc, pH 4.5, containing 0.2 mM ZnCl₂, 0.02% sodium azide and 0.5 U Jack bean α-mannosidase (JBAM) or buffer and incubated for 24 h at 37°C. Hydrophilic headgroups of each indiviual glycolipid were analysed by Biogel P4-gelfiltration prior and after JBAM treatment. Samples were mixed with 150 µl partially hydrolysed dextrane (150 mg/ml, prepared according to Yamashita *et al*., Meth. Enzymol. 83, 105-126, 1982) and loaded to 140x1 cm columns of Biogel P4 (Biorad) equilibrated and eluted with 200 mM ammonium acetate. Standard detection was performed by orcinol staining as described (Tomavo *et al*., 1992). Out of six different glycolipids four sizetypes of hydrophilic headgroups (9.5, 8.5, 7 and 6 GU respectively) could be detected. JBAM has a broad specificty for any terminal, non-substituted α-D-mannose residue. Headgroups derived from glycolipids I, II and III were in contrast to those derived from glycolipids IV, V and VI insensitive to mannosidase treatment which together with the labeling data indicates the presence of ethanolamine phosphate bound to the terminal mannose of glycolipid I, II and III.

In order to isolate the core glycans of the GPI species, [³H]glucosamine labeled TLC purified glycolipids were dephosphorylated, deaminated and reduced essentially according to methods previously described (Mayor *et al*., Methods in Enzymol., 1, 297-305, 1990). Briefly, dephosphorylation was performed by incubation with 48% aqueous HF for 60 h at 0°C and stopped by neutralisation with frozen LiOH. The reaction mixture was desalted, deaminated and reduced as described above, and finally desalted over 1 ml of AG50W-X12 and a tandem ion-exchange column of 0.4 ml of Chelex 100 (Na⁺), 0.2 ml of AG50W-X12 (H⁺), 0.4 ml AG3-X4 (OH⁻) and 0.2 ml of QAE-Sephadex (OH⁻) and filtered through a 0.25 µm filter. Core glycans were analysed by Bio-Gel P4 gelchromatography, Dionex HPAEC and lectin affinity chromatography.

Radiolabeled core glycans were analysed on a Dionex Bio Liquid Chromatograph (HPAEC). Samples were mixed with partially hydrolysed dextran prior to injection to a Carbopak PA1 column equilibrated with 100 mM NaOH. Elution was accomplished using 100% Buffer A (100 mM NaOH) for 6 min, followed by a linear increase of buffer B (100 mM NaOH, 0,25 M NaOAc) from 0 to 30% in 30 min at a flow rate of 1 ml/min. Fractions of 0.4 ml were collected. Core glycans from all six glycolipid species coeluted with the 3 DU (dextran unit, here Glc₃) standard. This elution position clearly differs from 2.5 DU elution position of the simple Man3-AHM core glycan and is suggestive for a substitution of the *T*. *gondii* GPIs' glycans.

Radiolabeled core glycans derived from TLC purified GPIs were subjected to JBAM treatment as described above and desalted samples were then reanalysed by Dionex-HPAEC. The elution position of the major reaction product was 2.4 DU for all six glycolipids. This elution position is consistent with an only limited sensitivity of the glycan towards JBAM (unsubstituted core glycan elutes as 0.9 DU after JBAM treatment) due to a blocking non-mannose substituent linked to the trimannosyl core. To investigate the nature of this substituent a monosaccharide analysis of radiolabeled core glycans was performed. Core glycans derived from [³H]glucosamine labeled glycolipid II an III were dried and hydrolysed with 4 N HCl for 4 h at 100°C. The cooled reaction mixture was dried and flash evaporated twice with methanol 5% acetic acid and twice with methanol. The hydrolysates were mixed with a monosaccharide standard mixture containing 10 nmol of each monosaccharide and were analysed by Dionex-HPAEC using isocratic elution with IS mM NaOH at a flow rate of 1 ml/min and fractions of 0.25 ml were collected. Elution positions of individual monosaccharide standards were detected using pulsed amperiometric detection. Two radiolabeled monosaccharides were identified, anhydromannitol and galactosamine. Anhydromannitol is the derivate of the GPIs' deaminated and reduced non acetylated glucosamine. The finding of galactosamine proposes N-acetylgalactosamine (GalNAc) as a further component of the GPIs' core glycan.

In order to demonstrate the presence of terminal GalNAc residues, core glycans from all 6 glycolipid species were incubated with β-N-acetylhexosaminidase an exoglycosidase cleaving terminal β-bound N-acetylhexosamines. Briefly, samples were resuspended in 100 µl 50 mM sodium citrate pH 4.5 containing 0.5 U recombinant enzyme (New England Biolabs), incubated for 12 h at 37°C, boiled, desalted and analysed by Dionex-HPAEC as described above for core glycans. Core glycans from Glycolipid III and VI were sensitive to hexosaminidase treatment. Two fragments could be identified after treatment by HPAEC coeluting with GalNAc (1 DU) and Man3-AHM (2,5 DU) providing further evidence for the presence of N-acetylgalactosamine on these glycans. To show that GalNAc was the substituent blocking mannosidase activity a sequential treatment with hexosaminidase and JBAM was performed. HPAEC analysis of glycans from glycolipid III and VI treated this way resulted in two peaks coeluting with GalNAc and anhydromannitol showing complete mannosidase sensitivity of the glycan after removel of the GalNAc residue. In contrast core glycans derived from glycolipids I, II, IV and V were insensitive toward hexosaminidase treatment although n-acetylgalactosamine has been shown to be present by monosaccharide analysis.

Biogel P4-gelfiltration analysis of core glycans showed a size heterogeneity of core glycans. The core glycans derived from glycolipid III and VI coeluted with a Man3GalNAc-AHM standard form rat brain Thy 1 antigen membrane anchor and the 6 GU standard (to be referred to as core glycan A). Glycans from the hexosaminidase insensitive glycolipid species I, II, IV and V coeluted with a 7 GU standard (to be referred to as core glycan B). These findings show the presence of an additional substituent to the GalNAc residue hindering enzymatic removel of GalNAc.

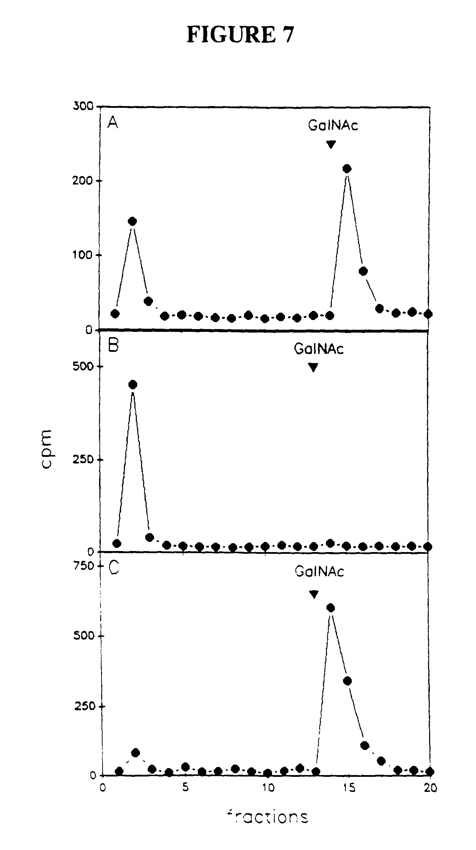

These data were confirmed by lectin-affinity-chromatography using *Wistaria floribunda* agglutinin a lectin specific for terminal β1,4 linked GalNAc. Lectin affinity chromatography was performed as previously described (Nakano *et al*., Arch. Biochem. Biophys. 311, 117-126, 1994). Briefly, radiolabeled core glycans derived from glycolipid II, III (core glycan A and B respectivly) and rat brain THY1 membrane anchor were applied to a 1 ml column of *W*. *floribunda* agglutinin agarose (5 mg lectin/ml gel) equilibrated in 50 mM Tris-HCl, pH 7.4 containing 0.02% sodium azide. Elution was accomplished using 25 ml of equilbration buffer followed by 15 ml of the same buffer containing 100 mM N-acetylgalactosamine as specific inhibitor. Core glycan A and the Man3GalNAcAHM-standard bound to the column and had to be eluted with the specific inhibitor GalNAc (Figure 7), whereas core glycan B showed no affinity towards the lectin showing that in these species GalNAc, although present. is not accesible to the lectin due to a further substituent!

### Example 6

### Identification of glucose as component of core glycan B by in vitro labeling using a tachyzoite cell free system

The experimental approach of in vitro labeling can overcome problems frequently encountered with in vivo labeling of tachyzoites in cell culture. These problems are mainly inefficient transport of the label through the various membrane systems surrounding the intracellular parasite (host cell plasmalemma, parasitophorous vacuole and tachyzoite membrane) and/or excessive catabolic metabolisation or epimerisation of the labeled sugar, all leading to poor and non-specific labeling. Here in vitro labeling was used to show glucosylation of *T. gondii* GPIs.

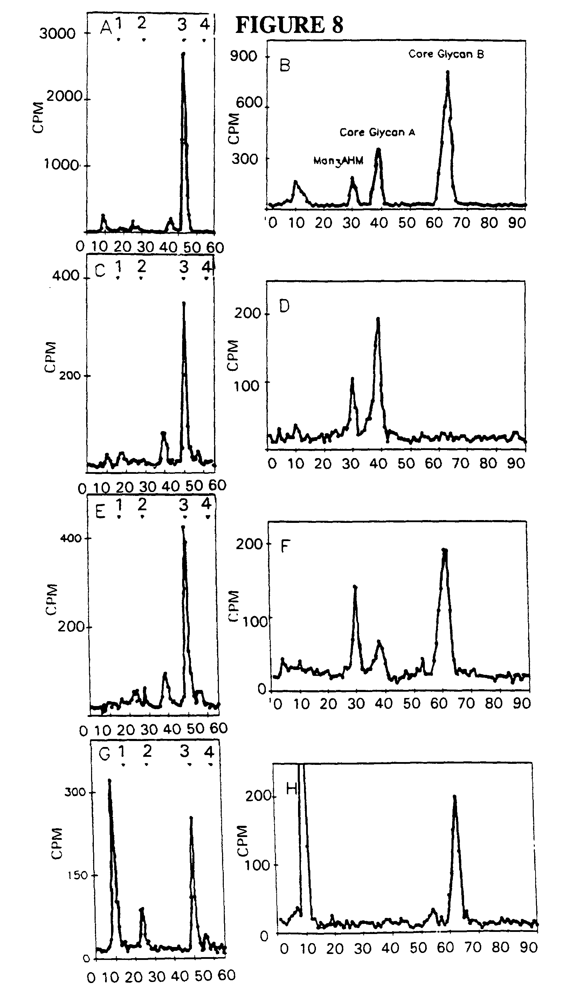

Tachyzoites were isolated from infected Vero cell cultures and purified from host cell membranes by glas wool filtration as described above. A cell free system for *in vitro* labeling was prepared with modifications according to the protocol of Masterson *et al*. (Cell 56, 793-800, 1989). Briefly, 2x10⁹ tachyzoites were hypotonically lysed in 375 µl icecold water containing 1 µg/ml leupeptin and 0.1 mM tosyllysinechloromethylketone (TLCK) for 5 min on ice and then homogenized by 50 strokes in 3 ml Dounce homogenizer. The lysate was mixed with an equal volume of double concentrated incubation buffer (50 mM Na HEPES pH 7.4, 25 mM KCl, 5 mM MgCl₂, 0.1 mM TLCK and 1 µg/ml leupeptin) and again briefly homogenized. 75 µl of this tachyzoite cell free membrane preparation representing 2x10⁸ tachyzoites were added to each incubation tube (containing predried nucleotide sugars, CoA and ATP see below) and was incubated for 2 h at 37°C. Incubation tubes contained 1 mM ATP, CoA, UDP-GlcNAC, UDP-GalNAc and GDP-Man (in the case of label other than GDP-3H-Man) and 2 µCi GDP-3H-Man or 5 µCi UDP-3H-Gluc. Incubation was terminated by addition of 2 ml CM and extraction of GPIs was subsequently performed as described above. Core glycans were prepared from glycolipids contained in the butanol phase by dephosphorylation, deamination and reduction as described above. Resulting core glycans were analysed by Dionex-HPAEC and aminopropyl-HPLC (Figure 8) (see example 5 for experimental details).

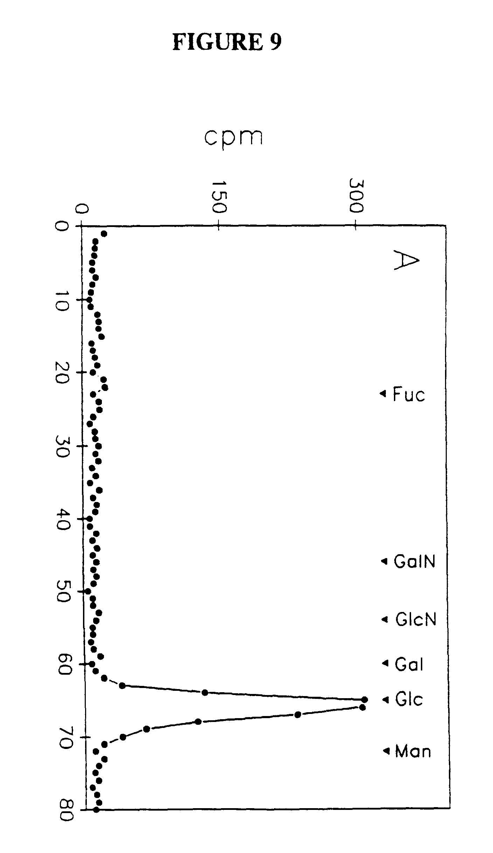

*In vitro* labeling with GDP-3H-Man resulted in the formation of glycolipids containing core glycans containing Man3AHM and core glycan A (Man3(GalNAc)AHM) as previously shown (Tomavo *et al*., 1992) core glycan B is not synthesized under these conditions (Figure 8D). In contrast labeling with UDP-3H-Glc resulted in a aminopropyl-HPLC peak coeluting with core glycan B (Figure 8H) and no labeling of core glycan A and Man3AHM. Core glycan B labeled via UDP-3H-Glc was purified by aminopropyl-HPLC. Purified labeled glycan was dried to a reacti vial™ and hydrolysis was accomplished by addition of 200 µl of 2 N trifluoracetic acid and incubation for 4 h at 100°C. The hydrolysate was desalted and subjected to Dionex-HPAEC monosaccharide analysis as described above. All radioactivity coeluted with the glucose standard (Figure 9). This data show that the additional substituent in core glycan B is glucose. To confirm this observation a stimulation experiment with unlabeled UDP-Glc was performed. A cell free system of tachyzoite membranes prepared as described above was supplemented with 1 mM UDP-Glc and labeled with GDP-3H-Man. Analysis of core glycan prepared under these conditions showed aside from the presence of Man3AHM and core glycan A also the formation of core glycan B (Figure 8F).

### Example 7

### Elucidation of complete structures of core glycan A and B

The strategy chosen for structural elucidation of *T. gondii* GPI core glycans was:
1. isolation of glycolipids from large numbers of tachyzoites by extraction and butanol/water phasepartition,
2. preparation of core glycans by dephosphorylation, deamination and reduction from the all six glycolipids containing butanol phase,
3. preparative HPLC-purification of core glycan A and B on an amino-propyl phase, and
4. extensive analysis of purified glycans by methanolysis/GC, FAB-MS, GC-MS and NMR.

Since the core glycan A and B coelute on Dionex-HPAEC columns and are imperfectly resolved on Bio-Gel P4, large quantities of individual glycan species were purified to homogeneity by separating the sample on aminopropyl-HPLC.

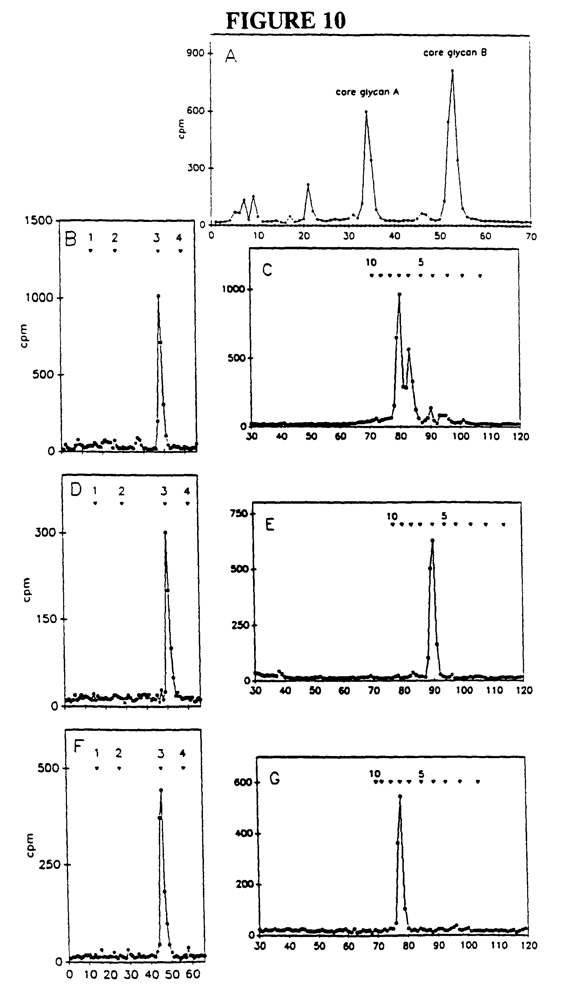

The core glycans were analysed and purified using a Waters 510 HPLC system. To establish the separation system radiolabeled core glycans were injected in 50 µl water to a Lichrosorb-NH2 column (4.9x250 mm, Merck) equilibrated and eluted isocraticaly with 28% water 72% acetonitrile at a flow rate of 1 ml/min. Fractions of 1 ml were collected and the elution positions of the individual glycans were identified by scintillation counting (Figure 10A). Fractions comprising peak A and B were pooled and analysed by using Bio-Gel P4 gel chromatography (Figure 10 E,G) and Dionex-HPAEC (Figure 10 D,F). These experiments showed that peak A was identical with core glycan A (coeluting with the 6 GU standard on Biogel P4) and peak B with core glycan B (coeluting with the 7 GU standard).

To obtain large amounts of radiolabeled core glycans to be used as tracer throughout the purification of unlabeled material chemical radiolabeling was performed. Glycolipids obtained from 2x10⁹ were dephosphorylated and deaminated as described above. The pH was adjusted to 10.5 and radiolabeling was performed by addition of 5 mCi NaB[³H]H₄ in 10 µl 0.1 N NaOH and incubation for 30 min. After acidification with acetic acid the sample was desalted as described above. Solid phase extraction on a 1 ml aminopropyl Sep Pak™-cartridge (Millipore-Waters) was applied to remove radiochemical impurities. The cartridge was prewashed with 8 ml water, 2 ml ethanol and 5 ml butanol using a syringe. The sample was taken up in 40 µl water, mixed with 2 ml butanol and applied to the cartridge, which was then eluted using 2 ml butanol, 4 ml butanol/ethanol (2:1), 18 ml ethanol and 3 ml water. Purified radiolabeled core glycans were recovered in the water. This material was analysed in parallel with core glycans generated from metabolically labeled GPI by Dionex-HPAEC, Bio-Gel P4 and aminopropyl-HPLC. These analysis showed that pure radiolabelled material (1.5x10⁷ cpm) identical to glycans derived from metabolically labeled glycolipids was obtained starting with 2 nmol of glycan.

For preparative purposes 25 nmol core glycans were mixed with 50,000 cpm radioactive tracer each run. Core glycans from 1.5 10¹¹ tachyzoites were purified by HPLC yielding roughly 30 nmol of glycan A and 150 nmol of glycan B. This material was further analysed by monosaccharide analysis and different mass spectrometry and NMR techniques.

Methanolysis was performed as described (Ferguson, GPI membrane anchors: Isolation and analysis; in Glycobiology. A practical approach, eds. Fukuda and Kobata, IRL Press Oxford Univ. Press. Chapter 8, pp. 349-383, 1993). Briefly, samples containing core glycan A and B respectivly derived from 2x10¹⁰ tachyzoites were dried and resuspended in dry methanol containing 1 N HCl and incubated for 24 h at 85°C. Cooled reaction mixtures were neutralised with Ag₂CO₃ and then re-N-acetylated by addition of 25 µl acetic anhydride and incubation for 15 h. After centrifugation the supernatent was dried in a speed vac™ (Savant) and derivatisation was accomplished by addition 40 µl of freshly prepared pyridine/hexamethyldisilazane/trimethylchlorosilane (Pierce) 5:1:1 and reaction for 30 min. Derivatized monosacharides were analysed using a Hewlett Packard 5890 gaschromatograph equiped with a 25 m x 0.32 mm Fused Silica CP-SIL-5CB column. The initial oven temperature of 130°C was raised to 220°C with 4°C/min and detection was by flame ionisation.

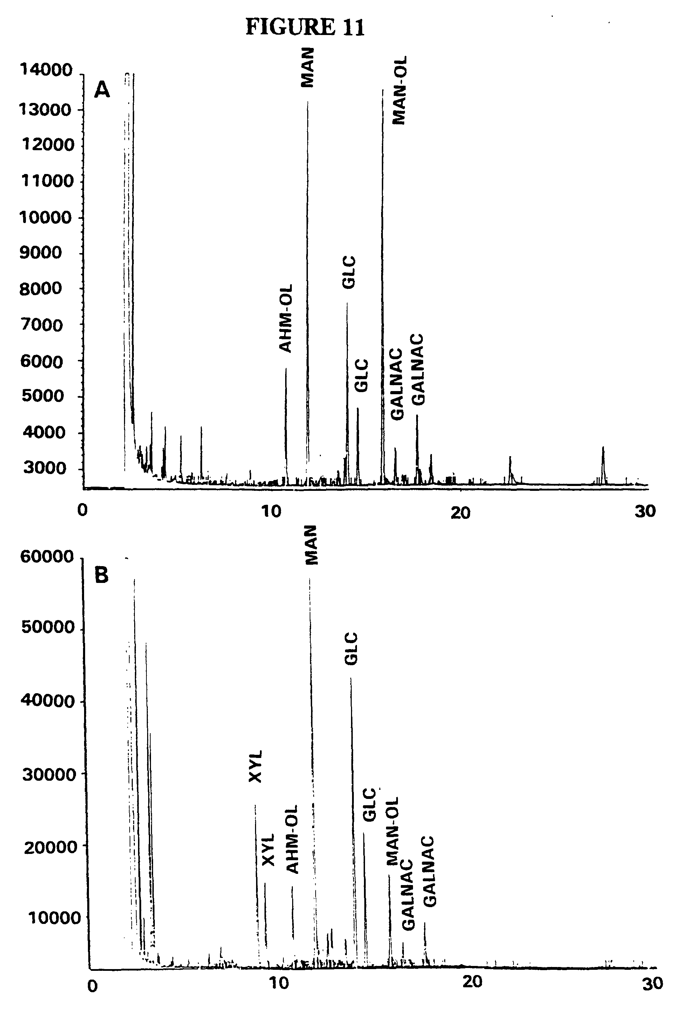

For both samples mannose, N-Acetylgalactosamine and 2,5 anhydromannitol were detected (Figure 11) in a rounded ratio of 3:1:1 indicating the presence of a trimannosyl core structure substituted by GalNAc. 2.8 mol xylose and 2 mol glucose per mol GalNAc was identified in the sample containing core glycan B and 1.3 mol of glucose in the sample containing core glycan A. Both sugars are frequent contaminations in monosaccharid analysis but the absence of any other monosaccharide difference between A and B supposes either xylose or glucose as substituent of GalNAc present in core glycan B. To adress this question mass spectrometry was applied.

Prior to analysis by Fast atom bombardement mass-spectrometry (FAB-MS) core glycan samples were permethylated according to the method of Ciucanu & Kerek (Carbohyd. Res. 131, 209, 1984) as described by Ferguson *et al*. (Science 239, 753, 1988). Briefly, samples were dried and resuspended in 100 µl dimethylsulfoxyde (Pierce) containing 120 mg/ml powdered NaOH. Under constant stirring three times 20 µl of methyliodine (Sigma) were added with 10 min incubation after each addition. Reaction was quenched with sodium thiosulfate and permethylated glycans were extracted with chloroform washed with water and concentrated. Samples were resuspended in 5 µl methanol and loaded into a matrix of glycerol. Spectra were obtained using a Finnigan MAT 90 mass spectrometer fitted with a WATV caesiumgun (Wagner Analysen Technik) operated with 22 kV and 2 µA.

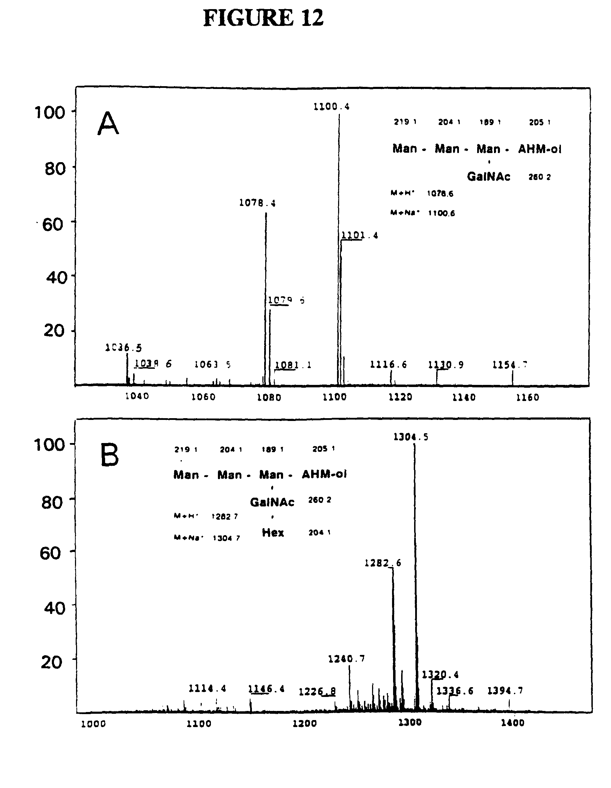

The FAB spectrum recorded (Figure 12) for permethylated core glycan A showed an molecular ion of m/z 1078.4 and the respective sodium adduct of m/z 1100.4. This mass is consistent with the structural proposal of Man-Man-(GalNAc)Man-AHM for glycan A. Analysis of glycan B resulted in detection of an M⁺Na⁺ ion of m/z 1282.7 and mlz 1304.5 for the respective M⁺Na⁺ ion. The mass difference between permethylated core glycan A and B is therefore 204.1 D which accounts exactly for an additional hexose residue on glycan B (Dell *et al*., Glycobiology, Fukuda & Kobata (eds.) Oxford, 187-222, 1993). Analysis of underivatised glycans by MALDI-TOF mass spectrometry confirmed this findings (data not shown).

Core glycan A and B isolated and purified from 10¹¹ tachyzoites was subjected to nuclear magnetic resonance spectroscopy (NMR) to confirm and define composition, anomery and lineage types. One-dimensional 500 mHz spectra were obtained as described by Hård *et al*. (Glycobiology, Fukuda & Kobata (eds.) Oxford, 221-242, 1993), 2D homonuclear Hartmann-Hahn (HOHAHA) spectra following the method described by Marion *et al*. (J. Mag. Reson. 85, 393-399, 1989) and 2D Rotated frame nuclear Overhauser effect (ROESY) according to Bothner-By *et al*. (J. Amer. Cem. Soc. 106, 811-813, 1984).

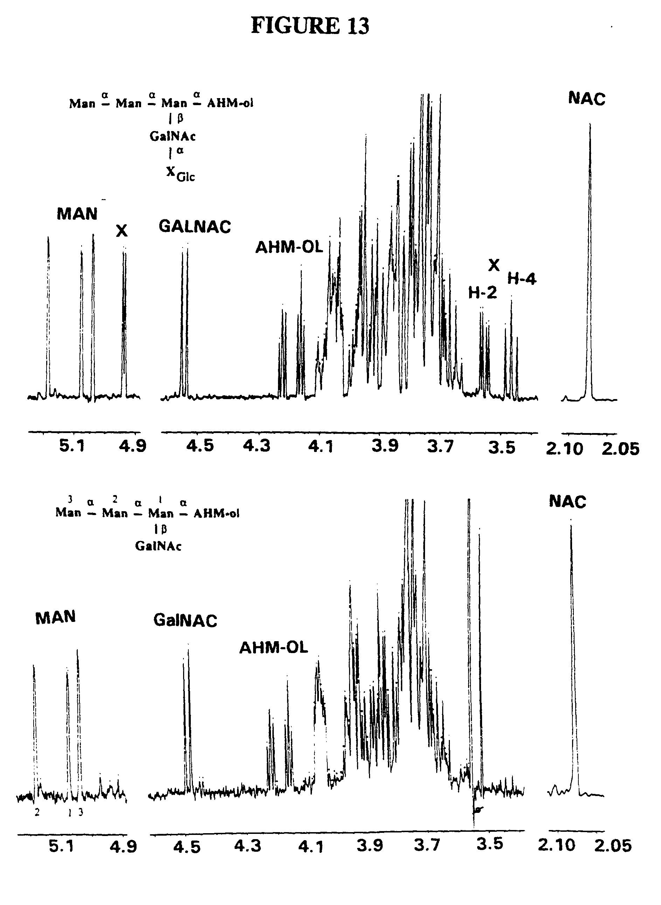

ID spectra obtained from core glycan A and B were interpreted in comparison to published spectra obtained for the *T. brucei* VSG (Ferguson *et al*., Science 239, 753-759, 1988) and the rat brain THY1 (Homans *et al*., Nature 333, 269-272, 1988) GPI membrane anchor and a data base. For core glycan A signals of anomeric protons from 3 α-bound mannose residues, anhydromannitol and one β-bound GalNAc were detected (Figure 13). Core glycan B showed an additional α-anomeric signal which in comparison with saccharose and maltose was identified as α-bound glucose. These findings were confirmed and extended on further protons using HOHAHA spectroscopy on core glycan B. The ppm values of identified protons for core glycan A and B are summarized in Table 1.

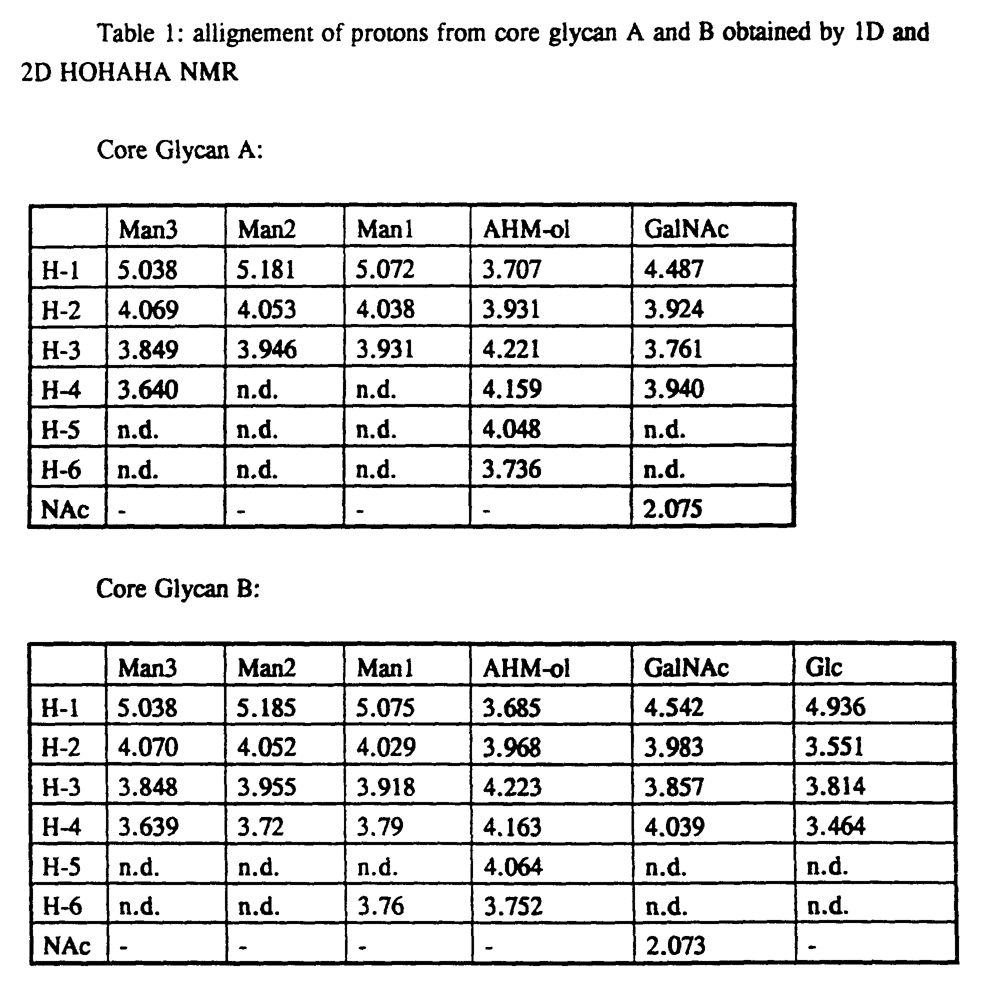

ROESY spectroscopy was applied on core glycan B to identify the carbon atoms of each monosaccharide residue involved in glycosidic linkages. Table 2 summarizes relevant cross peaks.

**Table 2:**

| ROESY cross peaks identifying glycosidic linkages in core glycan B | |
|---|---|
| Man 3 H-1/Man 2 H-2 | 5,038/4,052 |
| Man 2 H-1/Man 1 H-6 | 5,185/3,76 |
| Man 1 H-1/AHMol H-4 | 5,075/4,163 |
| GalNAc H-1/Man 1 H-4 | 4,542/3,79 |
| Glc H-1/GalNAc H-4 | 4,936/4,039 |

These data show that the mannose backbone of *T. gondii* core glycan A and B is identical to the previously described evolutionary conserved structure Manα1,2-Manα1,6-Manα1,4-AHM (McConville & Ferguson, Biochem. J. 294, 305-324, 1993). GalNAc is bound β-1,4 to the first mannose (proximal to AHM) and glucose is bound α1,4 to GalNAc in core glycan B. The structure of core glycan A resembles the structure of the nonmannosylated core glycan of the rat brain THY1 membrane anchor (Homans *et al*., Nature 333, 269-272, 1988) whereas core glycan B has a structure not yet described in the literature with α-bound glucose linked to GalNAc.

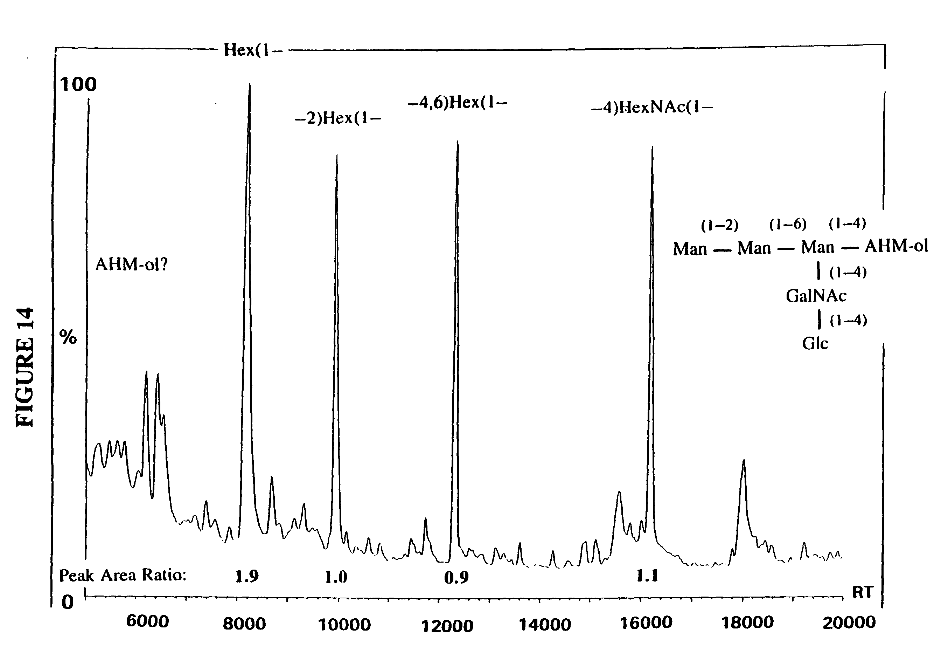

The data obtained by NMR spectroscopy on positions of glycosidic linkages were confirmed by methylation analysis performed according to a previously published procedure (Ferguson, Science 239, 753-759, 1988). Briefly, 80 nmol core glycan B were permethylated, acid hydrolysed and the resulting permethylated monosaccharides were reduced and acetylated. Following this procedure partial permethylated alditol actals (PMAA) are obtained which differ in their degree of methylation versus *O*-acetylation depending on the number and position of glycosidic linkages they were involved prior to hydrolysis. These PMAAs can be separated and quantified using gaschromatography-massspectroscopy (GC-MS). The total ion current chromatogram and mass spectra of individual peaks are shown in Figure 14. Four GC peaks were detected and identified by GC retention time and mass-spectrum as PMAAs of two terminal hexose residues (representing both the terminal mannose and glucose), one hexose linked at carbon 1 and 2, one hexose linked at carbons 1,4 and 6 and of a N-acetylhexosamine linked at carbon 1 and 4. The resulting structure is also given in Figure 14 and consistent with the data obtained by NMR and exoglycosidase sequencing.

All data obtained in the structural analysis of *T. gondii* free GPIs are consistent with the structure shown in Figure 15.

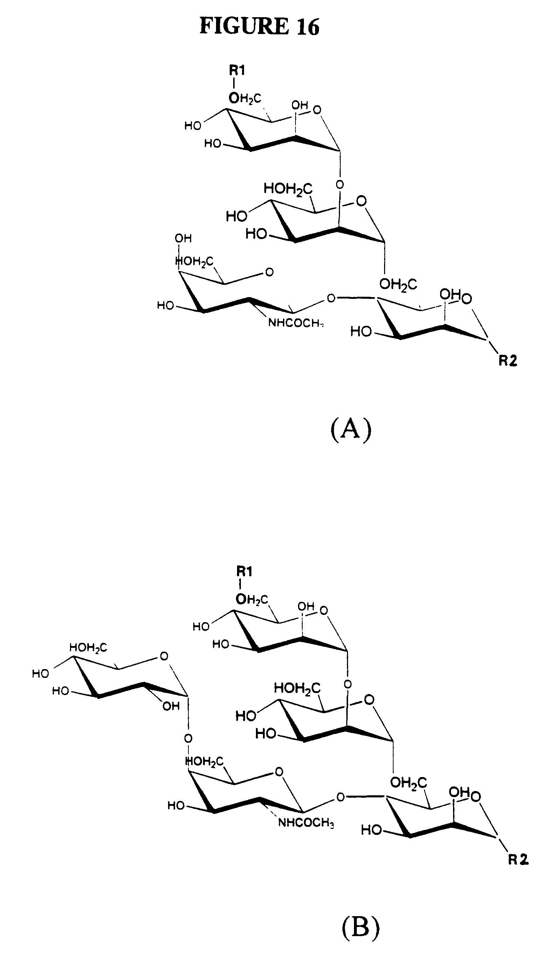

Structural representation of glycan core structures A and B are shown in Figure 16.

### Example 8

### Chemical synthesis and conjugation of glycoconjugate structures of core glycan A and B

### Synthesis:

Compounds A and B as well as analogues thereof can be synthesized by various routes. As an example the synthesis of pentasaccharide **13** is outlined. In the latter analogue of compound B, the non-essential anhydromannitol unit is replaced by a 3-aminopropyl linkage handle to facilitate conjugation of the pentasaccharide with macromolecular carriers. Alternatively, linkage handles having different chain lengths or containing other functional groups suitable for conjugation purposes (e.g. carboxylic acids, thiols or halogenides) may be used. The synthesis departs from mannose unit **1**, readily accessible by standard procedures from commercially available ethyl 1-thio-α-D-mannopyranoside (e.g. introduction of a 4,6-O-*p*-methoxybenzylidebe group with *p*-methoxybenzaldehyde dimethylacetal in the presence of *p*-toluenesulfonic acid, followed by benzylation of the resulting 2,3 diol with benzyl bromide and sodium hydride). Reaction of compound **1** with 3-N-benzyloxycarbonylamino-propan-1-ol (Berntsson *et al*., 1977, Acta Pharm. Suec., 14, 229) in the presence (Veeneman *et al*., 1990, Tetrahedron Lett., 31, 1331) of N-iodosuccinimide (NIS) and catalytic trifluoromethanesulfonic acid (TfOH) results in the formation of compound **2**. Compound **2** is treated (Johansson *et al*., 1984, J. Chem. Soc. Perkin Trans. I, 2371) with NaBH₃CN and dry CF₃COOH in (DMF) to give compound **3** having a selectively removable 6-O-*p*-methoxybenzyl (Mbn) function. Coupling of **3** with *N*-trichloroacetyl-(TCA) galactosamine derivative **4,** prepared by a similar procedure as reported for glucosamine (Blatter *et al*., 1994, Carbohydratye Res., 260, 189), in the presence of trimethylsilyl trifluoromethanesulfonate (TMSOTf) gives disaccharide **5.** Conversion of the trichloroacetyl (TCA) group into an acetyl group is accomplished with Bu₃SnH and 2,2'-Azobis(2-methylpropionitrile)(AIBN). Saponification of the three O-acetates gives access to the corresponding triol, the 4- and 6-OH functions of which can be selectively benzylated using a two-step procedure: 1) treatment with excess Bu₂SnO and 2) reaction with BnBr in the presence of CsF the resulting derivative is coupled at the 3-OH function with glucose unit **6** (Veeneman G.H., 1991, Thesis; Leiden University) in the presence of (NIS) and catalytic TfOH (Veeneman *et al*., 1990, Tetrahedron Lett., 31, 1331) to give trimer **7.** Treatment of **7** with 2,3-dichloro-5,6-dicyano-1,4-benzoquinone (DDQ)(Oikawa *et al*., 1982, Tetrahedron Lett., 23, 885) affords compound **8.** Reaction of **8** with mannose unit **9** (Elie *et al*., 1990, Tetrahedron, **46,** 8243) in the presence of NIS-TfOH gives tetramer **10,** the 2-O-benzoyl of which is removed by saponification with NaOMe to give **11**. Condensation of **11** with **9** then leads to pentasaccharide **12.** Finally, removal of the protecting groups with 1) NaOMe and 2) hydrogenation in the presence of 10% Pd on carbon gives target compound **13.**

### Conjugation:

Pentasaccharide **13** can be readily coupled to protein carriers as described (Evenberg *et al*., 1992, J. Infect. Dis., 165, S152-S155). First, **13** is reacted with commercially available hydroxysuccinimide ester of acetylthioacetic acid to give **14.** Compound **14** is then coupled with bromoacerylated protein (e.g. BSA, KLH, Tetanus toxoid) in the presence of hydroxylamine to afford conjugate **15**. Alternatively, **13** may be coupled directly with proteins in the presence of glutaric dialdehyde to give pentasaccharide-protein conjugates.

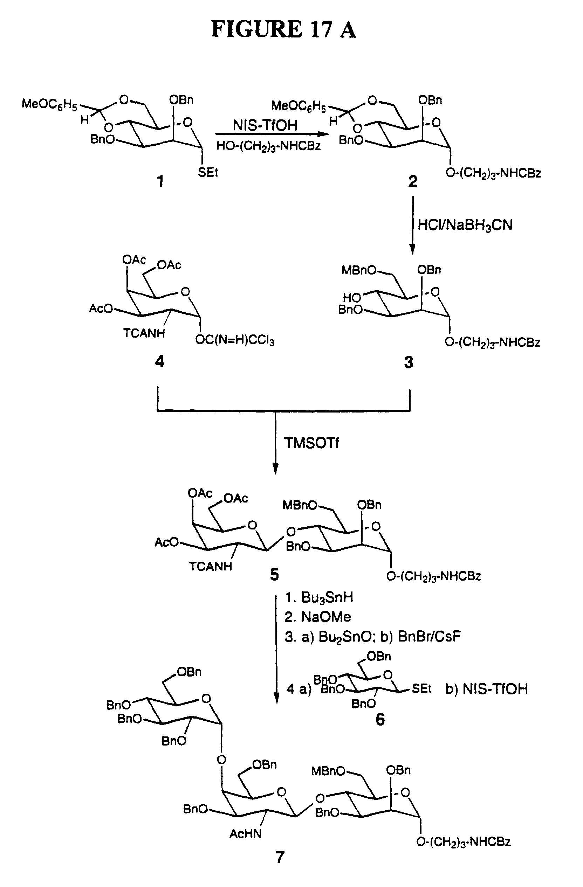

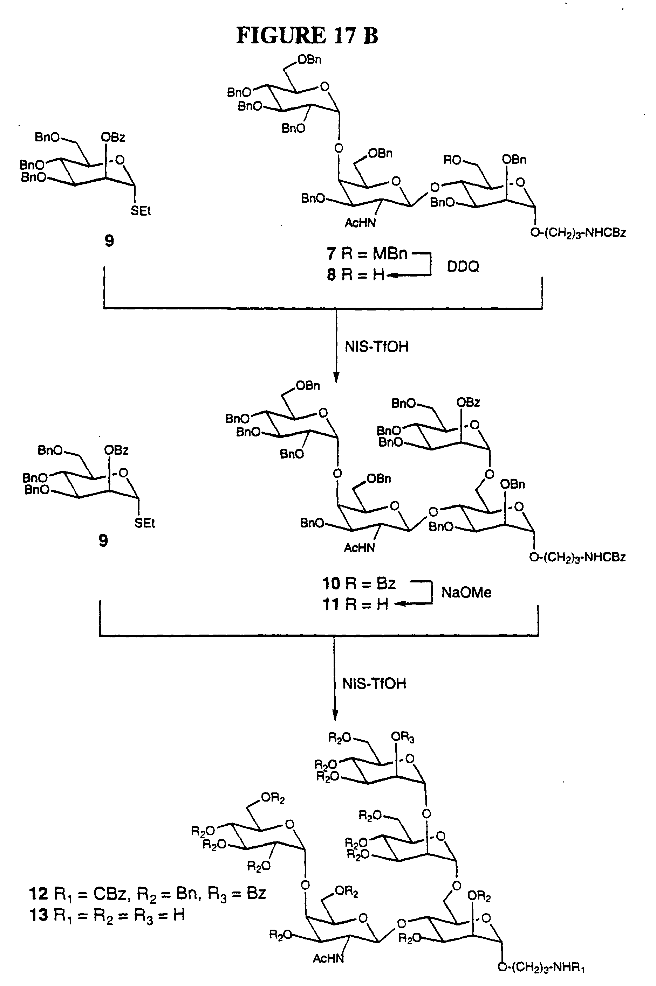

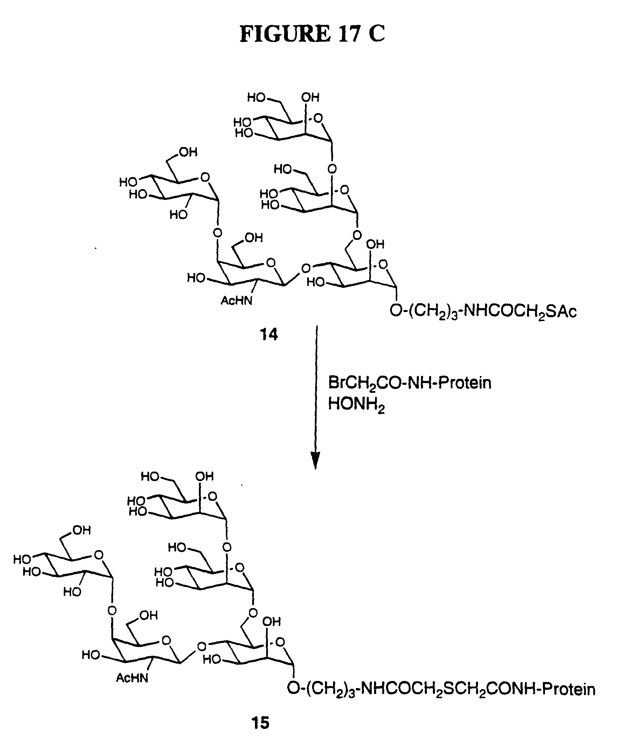

The above described synthesis and conjugation is exemplified in Figure 17 describing the reaction-schemes.

### Brief description of the figures:

**Fig 1. Antigen-specificity of mono- and polyclonal antibodies as demonstrated by Western blot analysis of total lysate of purified tachyzoites of *T*. *gondii*.** Filters were incubated with two human anti-Toxoplasma IgM monoclonal antibodies directed against glycolipid TOX.HuOT-1 and TOX.HuOT-2 (lane 1 and 2, respectively), two human anti- Toxoplasma IgM monoclonals, TOX.HuOT-3 and TOX.HuOT-8 as negative controls (lane 3, 4), mouse anti-glycolipid monoclonal antibodies T54E10 and T33F12 (lane 5 and 6, respectively), mouse anti-human chorionic gonadotrophin hormone (lane 7) and a pool of five human anti-Toxoplasma IgM fractions as positive control (lane 8).
**Fig 2: Western Blot analysis of *T*. *gondii* glycolipids (butanol phase):** Human seroconversion serum IgM (A) and IgG (B) and mouse monoclonal antibodies T33F12 (C) and T54E10 (D) (Tomavo *et al*., Parasitol. 108: 139-145, 1994)
**Fig 3: TLC analysis of *T. gondii* glycolipids:** Butanol phase derived from parasites metabolically labelled with ³H-glucosamine (A), ³H-palmitic acid (B), ³H-mannose (C) and ³H-etanolamine (D)
**Fig 4: Dot blot analysis of TLC purified *T. gondii* glycolipids:** TLC purified glycolipids were spotted to nitrocellulose and reacted with a pool of 5 human α-Tox IgM fractions and mouse α-glycolipid mAb T54E10.
**Fig 5: Dot blot analysis of TLC purified *T*. *gondii* glycolipids:** TLC purified glycolipids were spotted to nitrocellulose and reacted with human seroconversion sera
**Fig 6: Inhibition ELISA:** Inhibition ELISA using glycolipids contained in the butanol phase as antigen and mAb T54E10
**Fig 7: Analysis of core glycans derived from TLC purified glycolipids labelled with** ^{**3**}**H-glucosamine on Wistaria floribunda agglutinin agarose:** Core glycan A (A), core glycan (B), Man₄GalNAcAHM from rat brain Thy1 anchor (C), arrow indicates buffer change
**Fig 8: Dionex-HPAEC and aminopropyl-HPLC analysis of core glycans derived from glycolipids labeled *in vitro* using a tachyzoite cell free system: The** left row represents HPAEC- and the right row represents aminopropyl-HPLC analysis, A,B: core glycans derived from glycolipids labelled *in vivo*, core glycans derived from glycolipids labelled *in vitro* with GDP-3H-Man in the absence (C,D) and presence (E,F) of unlabelled UDP-Glc, G and F show core glycans derived from glycolipids labeled *in vitro* with UDP-3H-Glc
**Fig 9: HPAEC monosaccharide analysis of HPLC purified core glycan B labeled *in vitro* via UDP-3H-Glc:** Core glycan B was hydrolysed with 2 N TFA for 4h at 100°C. desalted and analysed by HPAEC using isocratic elution with 15 mM NaOH
**Fig 10: Preparative HPLC purification of core glycans directly from butanol phase:** Core glycans derived from glycolipids labelled with ³H-glucosamine (butanol phase) were separated on Lichrosorb NH₂ (A), equivalent material(B,C) and pooled fractions under peak A (D,E) and B (F,G) were analysed by HPAEC (left column) and Biogel P4 (right column), arrabic numbers indicate position of internal dextrane hydrolysate standard
**Fig 11: GC-monosaccharide analysis of core glycan samples:** HPLC fractions containing core glycan A (A) and B (B) were subjected to methanolysis, re-N-acetylation and TMS-derivatisation and analysed by GC
**Fig 12: Fast atom bombardement mass-spectroscopy (FAB-MS) of core glycans:** HPLC purified core glycan A (A) and B (B) were permethylated and analysed by FAB-MS
**Fig 13: 500 mHz** ^{**1**}**H-NMR spectra of core glycan A and B**
**Fig 14: Methylation analysis of core glycan B:** 80 nmol of core glycan B were permethylated, hydrolysed, reduced and acetylated, the resulting PMMAs were analysed by GC-MS. This figure represents the total ion chromatogram.
**Fig 15: Schematic representation of *T*. *gondii* glycoconjugate structure.** The structure represents glycoconjugate according to the present invention. A glycoconjugate can differ in presence or absence of terminal ethanolaminephosphate and/or their lipid moiety.
**Fig 16: Structure representation of *T. gondii* glycoconjugate structure.** The structure represents glycoconjugate according to the present invention, wherein A represents a core glycan wherein the unique glucose residue is absent and B represents a core glycan wherein the unique glucose residue is present. The R1 and R2 groups are described in the specification. A glycoconjugate can differ in presence or absence of terminal ethanolaminephosphate and/or their lipid moiety.
**Fig 17: Reaction schemes.** In the reaction schemes the synthesis and conjugation of glycoconjugates according to the present invention are shown in detail according to example 8.

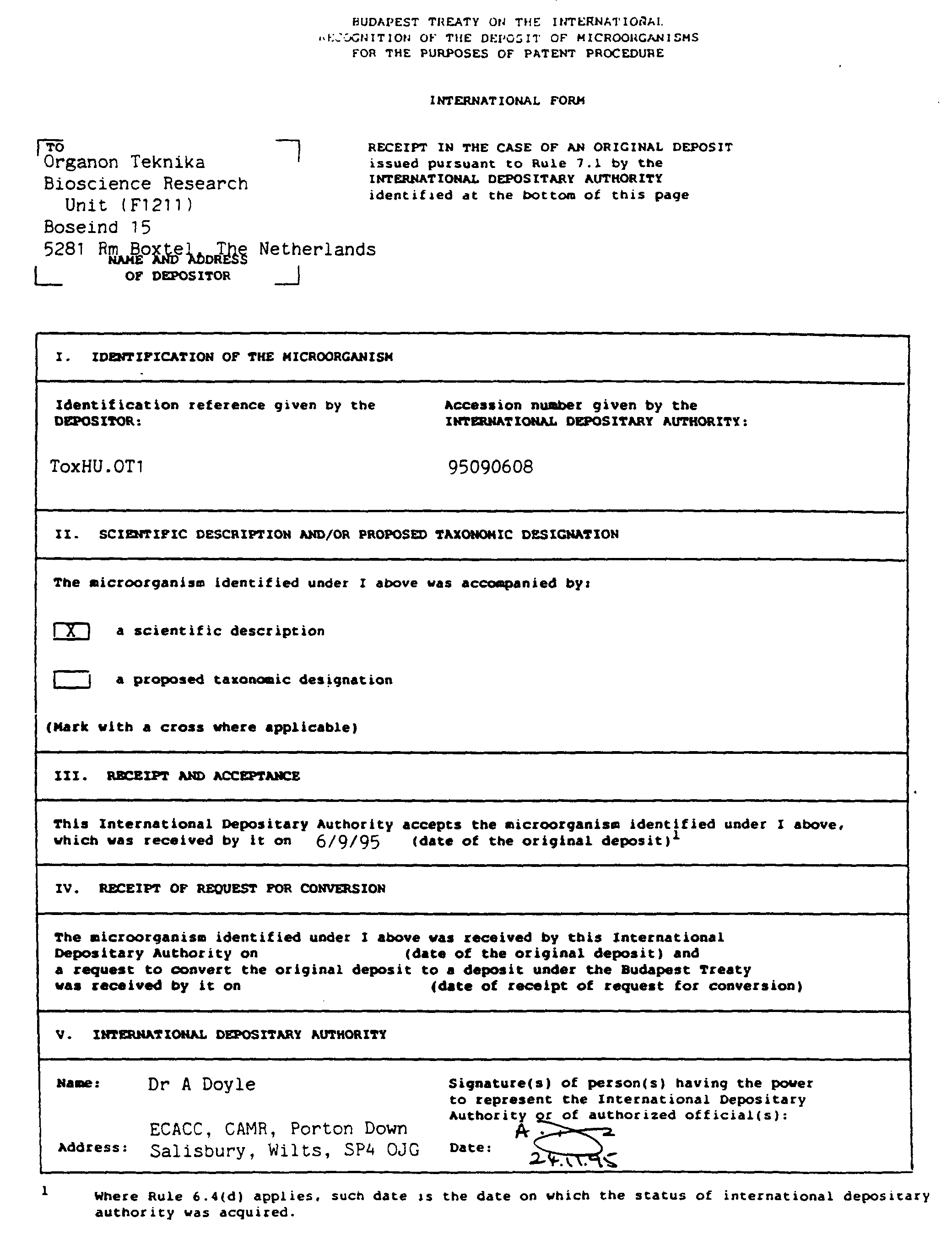
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO
Organon Teknika
Bioscience Research
Unit (F1211)
Boseind 15
5281 Rm Boxtel, The Netherlands
NAME AND ADDRESS
OF DEPOSITOR

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the
DEPOSITOR: | Accession number given by the
INTERNATIONAL DEPOSITARY AUTHORITY:

ToxHU.OT1 | 95090608

II. SCIENTIFIC DESCRIPTION AND/OR PROPOSED TAXONOMIC DESIGNATION

The microorganism identified under I above was accompanied by:

[X] a scientific description

[ ] a proposed taxonomic designation

(Mark with a cross where applicable)

III. RECEIPT AND ACCEPTANCE

This International Depositary Authority accepts the microorganism identified under I above,
which was received by it on 6/9/95 (date of the original deposit)1

IV. RECEIPT OF REQUEST FOR CONVERSION

The microorganism identified under I above was received by this International
Depositary Authority on (date of the original deposit) and
a request to convert the original deposit to a deposit under the Budapest Treaty
was received by it on (date of receipt of request for conversion)

V. INTERNATIONAL DEPOSITARY AUTHORITY

Name: Dr A Doyle | Signature(s) of person(s) having the power
to represent the International Depositary
Authority or of authorized official(s):
Address: ECACC, CAMR, Porton Down
Salisbury, Wilts, SP4 OJG | Date: 24.11.95

1 Where Rule 6.4(d) applies, such date is the date on which the status of international depositary
authority was acquired.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO
Organon Teknika
Bioscience Research
Unit (F1211)
Boseind 15
5281 Rm Boxtel
The Netherlands

NAME AND ADDRESS OF THE PARTY
TO WHOM THE VIABILITY STATEMENT
IS ISSUED

VIABILITY STATEMENT
issued pursuant to Rule 10.2 by the
INTERNATIONAL DEPOSITARY AUTHORITY
identified on the following page

I. DEPOSITOR | II. IDENTIFICATION OF THE MICROORGANISM
Name: Organon Teknika | Accession number given by the
Address: Bioscience Research Unit (F1211) | INTERNATIONAL DEPOSITARY AUTHORITY:
Boseind 15 | 95090608
5281 Rm Boxtel | Date of the deposit or of the transfer:
The Netherlands | 6/9/95

III. VIABILITY STATEMENT
The viability of the microorganism identified under II above was tested
on 6/9/95 2. On that date, the said microorganism was

[X]3 viable

[ ]3 no longer viable

1 Indicate the date of the original deposit or, where a new deposit or a transfer has been
made, the most recent relevant date (date of the new deposit or date of the transfer).

2 In the cases referred to in Rule 10.2(a)(ii) and (iii), refer to the most recent viability
test.

3 Mark with a cross the applicable box.

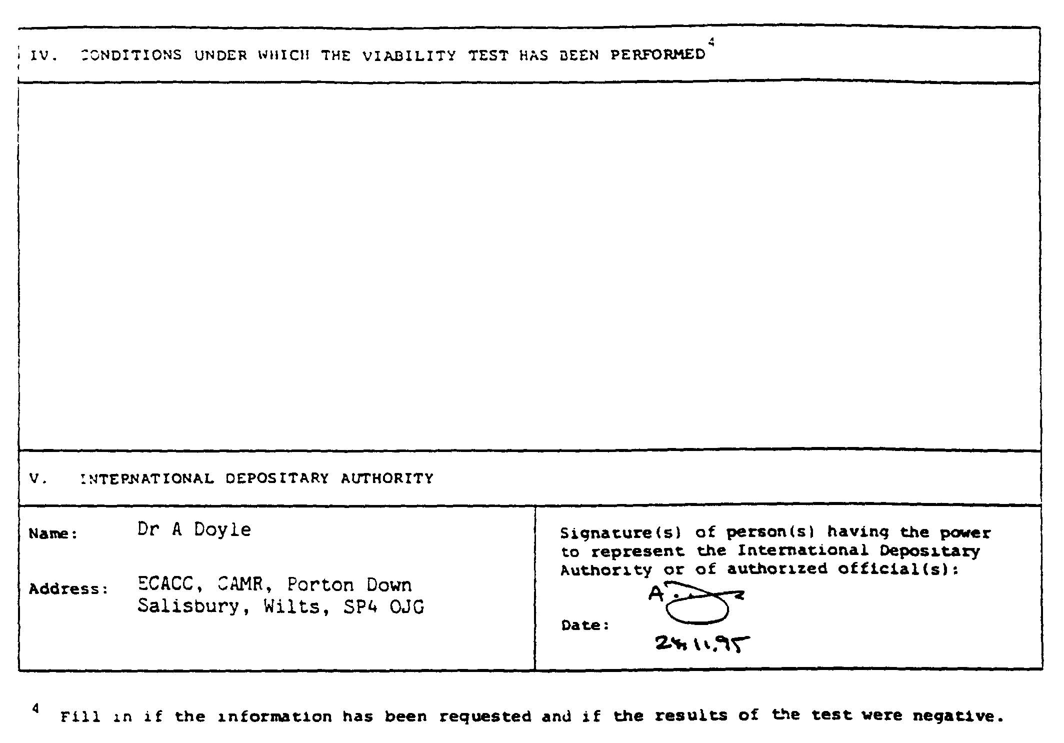
IV. CONDITIONS UNDER WHICH THE VIABILITY TEST HAS BEEN PERFORMED⁴

V. INTERNATIONAL DEPOSITARY AUTHORITY

Name: Dr A Doyle | Signature(s) of person(s) having the power
 | to represent the International Depositary
Address: ECACC, CAMR, Porton Down | Authority or of authorized official(s):
Salisbury, Wilts, SP4 OJG |
 | Date:
 | 24.11.95

⁴ Fill in if the information has been requested and if the results of the test were negative.

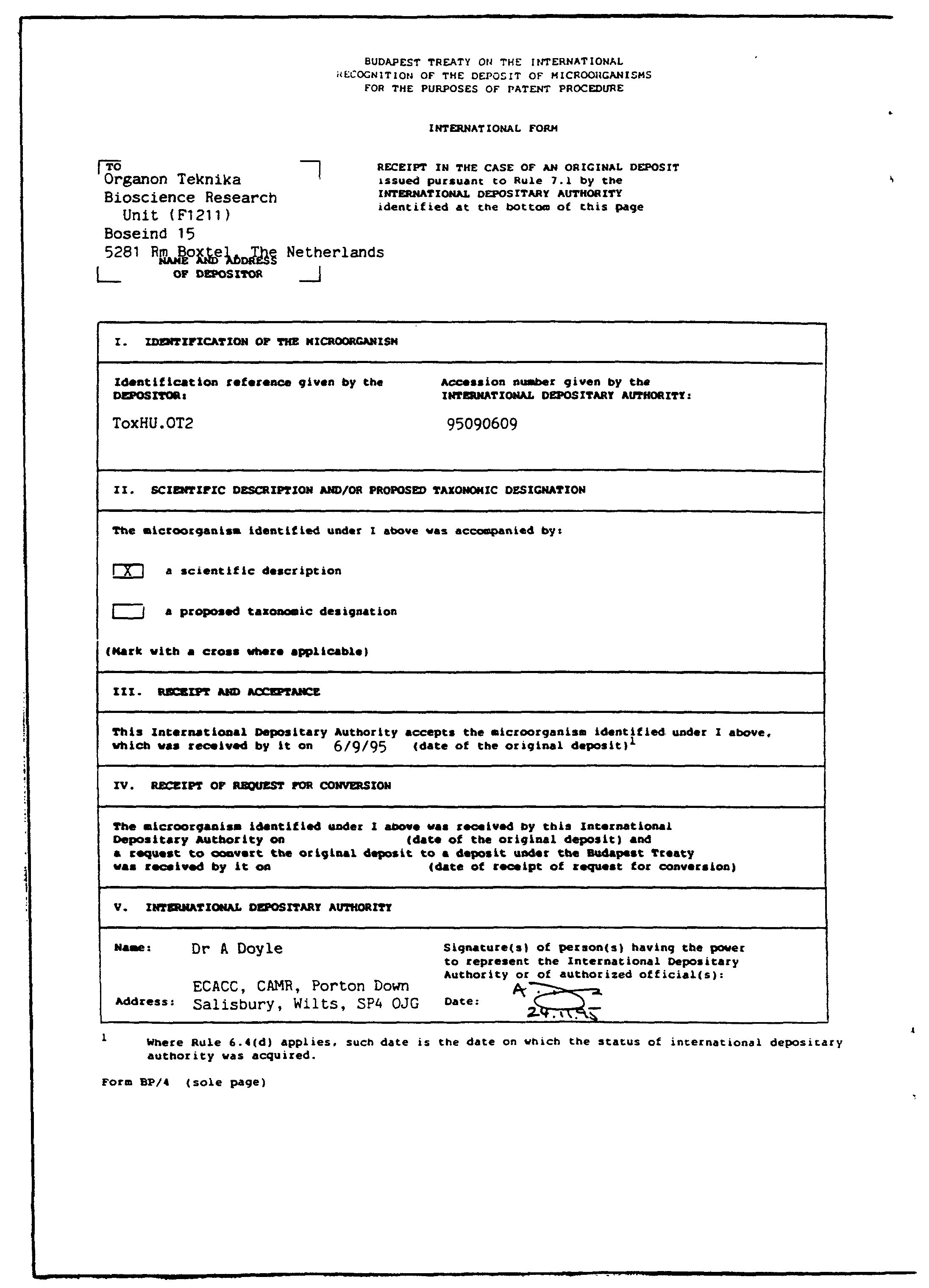
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO
Organon Teknika
Bioscience Research
Unit (F1211)
Boseind 15
5281 Rm Boxtel, The Netherlands
NAME AND ADDRESS
OF DEPOSITOR

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the
DEPOSITOR:
ToxHU.OT2

Accession number given by the
INTERNATIONAL DEPOSITARY AUTHORITY:
95090609

II. SCIENTIFIC DESCRIPTION AND/OR PROPOSED TAXONOMIC DESIGNATION

The microorganism identified under I above was accompanied by:

[X] a scientific description

[ ] a proposed taxonomic designation

(Mark with a cross where applicable)

III. RECEIPT AND ACCEPTANCE

This International Depositary Authority accepts the microorganism identified under I above,
which was received by it on 6/9/95 (date of the original deposit)1

IV. RECEIPT OF REQUEST FOR CONVERSION

The microorganism identified under I above was received by this International
Depositary Authority on (date of the original deposit) and
a request to convert the original deposit to a deposit under the Budapest Treaty
was received by it on (date of receipt of request for conversion)

V. INTERNATIONAL DEPOSITARY AUTHORITY

Name: Dr A Doyle

Address: ECACC, CAMR, Porton Down
Salisbury, Wilts, SP4 OJG

Signature(s) of person(s) having the power
to represent the International Depositary
Authority or of authorized official(s):
Date: 24.11.95

1 Where Rule 6.4(d) applies, such date is the date on which the status of international depositary
authority was acquired.

Form BP/4 (sole page)

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO
Organon Teknika
Bioscience Research
Unit (F1211)
Boseind 15
5281 Rm Boxtel
The Netherlands

NAME AND ADDRESS OF THE PARTY
TO WHOM THE VIABILITY STATEMENT
IS ISSUED

VIABILITY STATEMENT
issued pursuant to Rule 10.2 by the
INTERNATIONAL DEPOSITARY AUTHORITY
identified on the following page

I. DEPOSITOR | II. IDENTIFICATION OF THE MICROORGANISM
Name: Organon Teknika
Address: Bioscience Research Unit (F1211)
Boseind 15
5281 Rm Boxtel
The Netherlands | Accession number given by the
INTERNATIONAL DEPOSITARY AUTHORITY:
95090609
Date of the deposit or of the transfer:
6/9/95

III. VIABILITY STATEMENT
The viability of the microorganism identified under II above was tested
on 6/9/95 2. On that date, the said microorganism was
[X]3 viable
[ ]3 no longer viable

1 Indicate the date of the original deposit or, where a new deposit or a transfer has been
made, the most recent relevant date (date of the new deposit or date of the transfer).
2 In the cases referred to in Rule 10.2(a)(ii) and (iii), refer to the most recent viability
test.
3 Mark with a cross the applicable box.

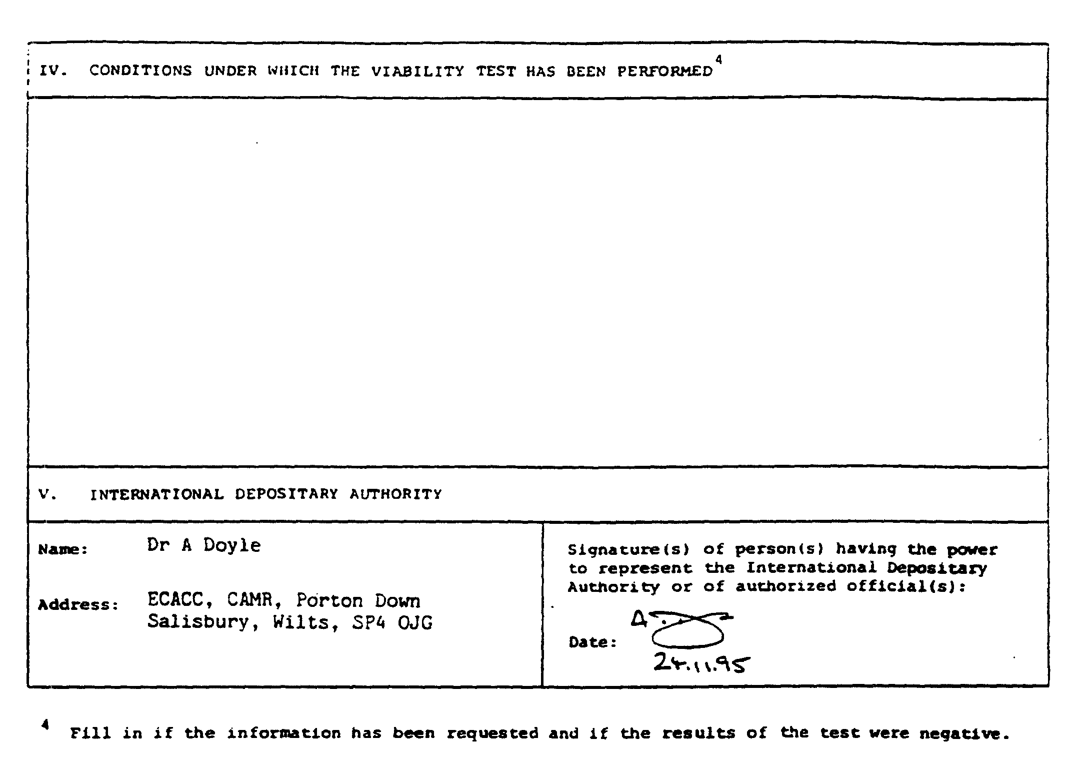
IV. CONDITIONS UNDER WHICH THE VIABILITY TEST HAS BEEN PERFORMED⁴

V. INTERNATIONAL DEPOSITARY AUTHORITY

Name: Dr A Doyle | Signature(s) of person(s) having the power
 | to represent the International Depositary
Address: ECACC, CAMR, Porton Down | Authority or of authorized official(s):
Salisbury, Wilts, SP4 OJG |
 | Date:
 | 24.11.95

⁴ Fill in if the information has been requested and if the results of the test were negative.

## Claims

1. Glycoconjugate comprising a glycan core structure having the general formula 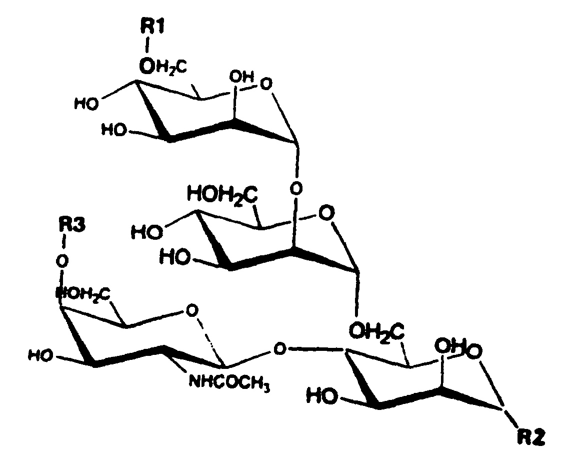 wherein R1 = Hydrogen, -PO₃-CH₂-CH₂-NH₂, or -PO₃-CH₂-CH₂-NH-X, wherein X= a *T. gondii* specific antigen;
wherein R2= α-O-R4, α(1-4)-O-anhydromannitol or α(1-4)-O-glucoseamine optionally α(1-6)-linked to the inositol moiety of a phosphatidylinositol membrane anchor;
wherein R3= a monosaccharide moiety,
wherein R4= Hydrogen or -L-R5, wherein L= a bivalent organic radical and
R5 = a functional group that allows coupling to a carrier.

2. Glycoconjugate according to claim 1, **characterized in that** said monosaccharide moiety is a hexose moiety.

3. Glycoconjugate according to claim 2, **characterized in that** said hexose moiety is a glucose moiety.

4. Glycoconjugate according to claim 3. **characterized in that** said glucose moiety is bonded to the GalNAc-moiety by an α-1,4 glycosidic bond.

5. Glycoconjugate according to any one of claims 1-4, **characterized in that** said glycoconjugate comprises the following Structure: 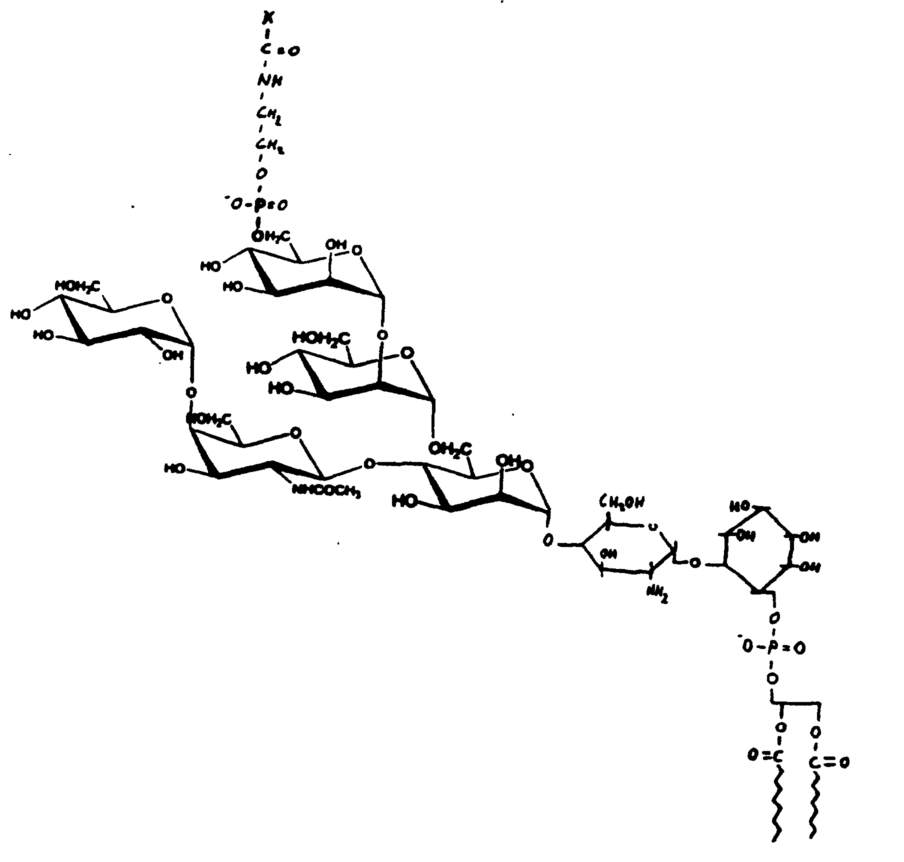 wherein X= a *T*. *gondii* specific antigen

6. Glycoconjugate according to any of claims 1-5. **characterized in that** said glycoconjugate is obtainable from *T*. *gondii* tachyzoites by an isolation procedure comprising the steps of:
- disruption of the tachyzoite membrane. and,
- extracting the resulting tachyzoite suspension with a chloroform/methanol/water mixture.

7. Glycoconjugate according to any of claims 1-6, **characterized in that** said glycoconjugate is immunoreactive with human monoclonal antibodies produced by the immortalized cell-line deposited with the European Collection of Animal Cell Cultures. Porton Down (UK), under deposit number 95090608 or 95090609.

8. Antibody directed against a *T*. *gondii* glycoconjugate. **characterized in that** said antibody is immunoreactive with the glycoconjugate according to any of claims 1-7.

9. Antibody according to claim 8, **characterized in that** said antibody is a monoclonal antibody.

10. Monoclonal antibody which binds to an epitope of a *T*. *gondii* glycoconjugate, which epitope is recognized by monoclonal antibody TOX.HuOT-1 or TOX.HuOT-2 produced by the hybridoma cell lines deposited with the European Collection of Animal Cell Cultures, Porton Down (UK), under deposit No. 95090608 and 95090609 respectively.

11. Monoclonal antibody TOX.HuOT-1 or TOX.HuOT-2 produced by the hybridoma cell lines deposited with the European Collection of Animal Cell Cultures, Porton Down (UK), under deposit No. 95090608 and 95090609 respectively.

12. Immortalized cell line capable of producing monoclonal antibodies according to any of claims 9-11.

13. Immortalized cell line deposited with the European Collection of Animal Cell Cultures. Porton Down (UK), under deposit No. 95090608 and 95090609 respectively.

14. Immunochemical reagent. **characterized in that** it comprises a glycoconjugate according to any of claims 1-7.

15. Immunochemical reagent, **characterized in that** it comprises an antibody according to any of claims 8-11.

16. A method for the detection of *T*. *gondii* in a test fluid, **characterized in that** said test fluid is contacted with an antibody according to any of claims 9-11 whereafter the presence of immune complexes is detected.

17. A method for the detection of antibodies directed against *T*. *gondii* in a test fluid, **characterized in that** an immunochemical reagent according to claim 14 is brought into contact with the test fluid and the presence of immune complexes formed in the test fluid is detected.

18. A method for the detection of *T. gondii* in a test fluid, **characterized in that** an immunochemical reagent according to claim 15 is brought into contact with the test fluid, whereto antibodies directed to *T*. *gondii* are brought into contact, and the presence of immune complexes formed is detected.

19. A test-kit for the detection of *T. gondii* infection, **characterized in that** said test-kit comprises an immunochemical reagent according to claim 14.

20. A test-kit for the detection of *T. gondii* infection. **characterized in that** said test-kit comprises an immunochemical reagent according to claim 15.

## Patentansprüche

1. Glykokonjugat, welches eine Glykankernstruktur mit der allgemeinen Formel 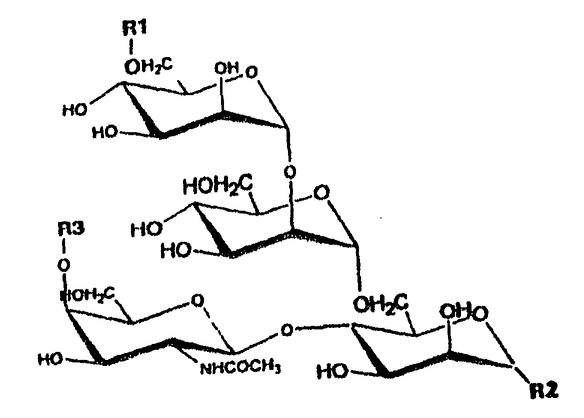 umfasst, worin R1 = Wasserstoff, -PO₃-CH₂-CH₂-NH₂ oder -PO₃-CH₂-CH₂-NH-X ist, worin X = ein *T. gondii* spezifisches Antigen ist;
worin R2 = α-O-R4, α(1-4)-O-Anhydromannitol oder α(1-4)-O-Glukosamin ist, das gegebenenfalls an den Inositol-Anteil eines Phosphatidylinositolmembranankers über α(1-6) gebunden ist;
worin R3 = ein Monosaccharid-Anteil ist,
worin R4 = Wasserstoff oder -L-R5 ist, worin L = ein bivalentes organisches Radikal und R5 = eine funktionale Gruppe ist, die das Koppeln an einen Träger erlaubt.

2. Glykokonjugat nach Anspruch 1, **dadurch gekennzeichnet, dass** der genannte Monosaccharid-Anteil ein Hexose-Anteil ist.

3. Glykokonjugat nach Anspruch 2, **dadurch gekennzeichnet, dass** der genannte Hexose-Anteil ein Glukose-Anteil ist.

4. Glykokonjugat nach Anspruch 3, **dadurch gekennzeichnet, dass** der genannte Glukose-Anteil mittels einer α-1,4-glykosidischen Bindung an den GalNac-Anteil gebunden ist.

5. Glykokonjugat nach einem der Ansprüche 1-4, **dadurch gekennzeichnet, dass** das genannte Glykokonjugat die folgende Struktur umfasst : 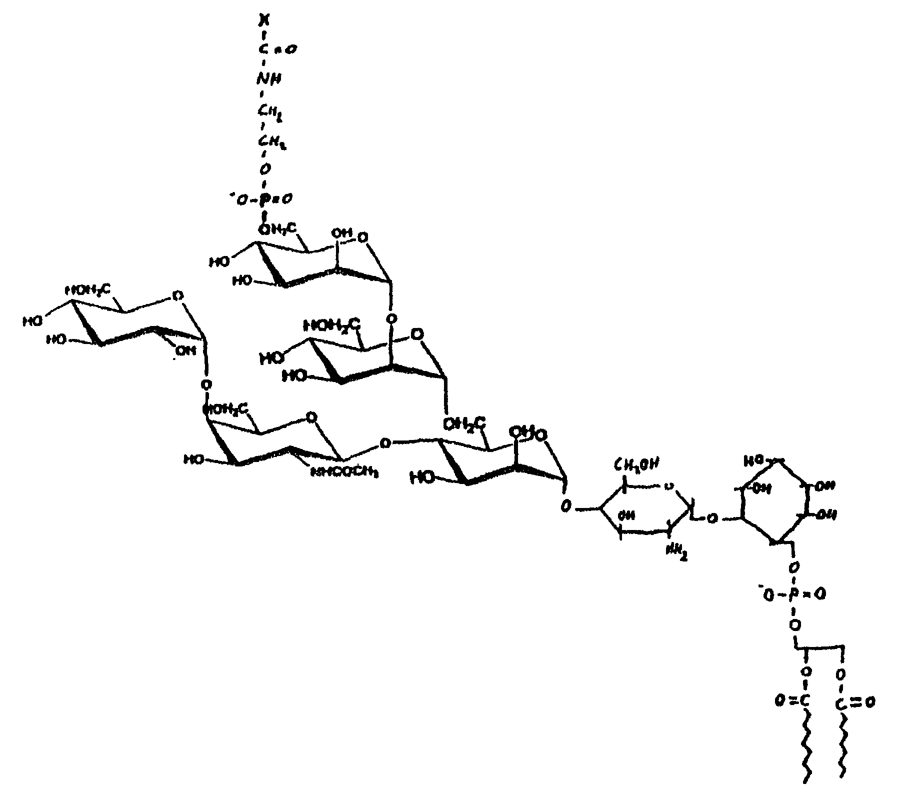 worin X = ein *T. gondii* spezifisches Antigen ist.

6. Glykokonjugat nach einem der Ansprüche 1-5, **dadurch gekennzeichnet, dass** das genannte Glykokonjugat von *T. gondii* Tachozoiten mittels eines Isolierungsverfahrens erhältlich ist, das die folgenden Schritte umfasst :
- Aufbrechen der Tachozoiten-Membran und
- Extrahieren der daraus resultierenden Tachozyten-Suspension mit einer Chloroform/Methanol/Wasser-Mischung.

7. Glykokonjugat nach einem der Ansprüche 1-6, **dadurch gekennzeichnet, dass** das genannte Glykokonjugat mit menschlichen monoklonalen Antikörpern immunreagiert, welche durch die bei der Europäischen Sammlung von Tierzellkulturen, Porton Down (GB) unter der Hinterlegungsnummer 95090608 oder 95090609 hinterlegte unsterbliche Zelllinie produziert werden.

8. Antikörper, der gegen ein *T*. *gondii*-Glykokonjugat gerichtet ist, **dadurch gekennzeichnet, dass** der genannte Antikörper mit dem Glykokonjugat nach einem der Ansprtiche 1-4 immunreagiert.

9. Antikörper nach Anspruch 8, **dadurch gekennzeichnet, dass** der genannte Antikörper ein monoklonaler Antikörper ist.

10. Monoklonaler Antikörper, der an ein Epitop eines *T*. *gondii*-Glykokonjugats bindet, wobei das Epitop von dem monoklonalen Antikörper TOX.HuOT-1 oder TOX.HuOT-2 erkannt wird, der von der Hybridomzelle produziert wird, die bei der Europäischen Sammlung von Tierzellkulturen, Porton Down (GB) unter den Hinterlegungsnummer 95090608 beziehungsweise 95090609 hinterlegt ist.

11. Monoklonaler Antikörper TOX.HuOT-1 oder TOX.HuOT-2, der von der bei der Europäischen Sammlung von Tierzellkulturen, Porton Down (GB) unter der Hinterlegungsnummer 95090608 beziehungsweise 95090609 hinterlegten Zelllinie pioduziert wird.

12. Unsterbliche Zelllinie, die fähig ist monoklonale Antikörper gemäss einem der Ansprüche 9-11 zu produzieren.

13. Unsterbliche Zelllinie, die bei der Europäischen Sammlung von Tierzellkulturen, Porton Down (GB) unter der Hinterlegungsnummer 95090608 beziehungsweise 95090609 hinterlegt ist.

14. Immunochemisches Reagenz, **dadurch gekennzeichnet, dass** es ein Glykokonjugat nach einem der Ansprüche 1-7 umfasst.

15. Immunochemisches Reagenz, **dadurch gekennzeichnet, dass** es einen Antikörper nach einem der Ansprüche 8-11 umfasst.

16. Verfahren zum Nachweis von *T*. *gondii* in einer Testflüssigkeit, **dadurch gekennzeichnet, dass** die genannte Testflüssigkeit mit einem Antikörper nach einem der Ansprüche 9-11 in Kontakt gebracht wird, wonach die Anwesenheit eines Immunkomplexes nachgewiesen wird.

17. Verfahren zum Nachweis von Antikörpern, die gegen *T. gondii* gerichtet sind, in einer Testflüssigkeit, **dadurch gekennzeichnet, dass** ein immunchemisches Reagenz gemäss Anspruch 14 mit der Testfflüssigkeit in Kontakt gebracht wird und die Anwesenheit von gebildeten Immunkomplexen in der Testflüssigkeit nachgewiesen wird.

18. Verfahren zum Nachweis von *T*. *gondii* in einer Testflüssigkeit, **dadurch gekennzeichnet, dass** das immunchemisches Reagenz gemäss Anspruch 15 mit der Testflüssigkeit in Kontakt wird, in dem gegen T. gondii gerichtete Antikörper in Kontakt gebracht werden und die Anwesenheit von gebildeten Immunkomplexen in der Testflüssigkeit nachgewiesen wird.

19. Test-Set zum Nachweis einer *T*. *gondii*-Infektion, **dadurch gekennzeichnet, dass** das genannte Test-Set ein immunchemisches Reagenz nach Anspruch 14 umfasst.

20. Test-Set zum Nachweis einer *T*. *gondii*-Infektion, **dadurch gekennzeichnet, dass** das genannte Test-Set ein immunchemisches Reagenz nach Anspruch 15 umfasst.

## Revendications

1. Glycoconjugué comprenant une structure centrale glycane de formule générale : 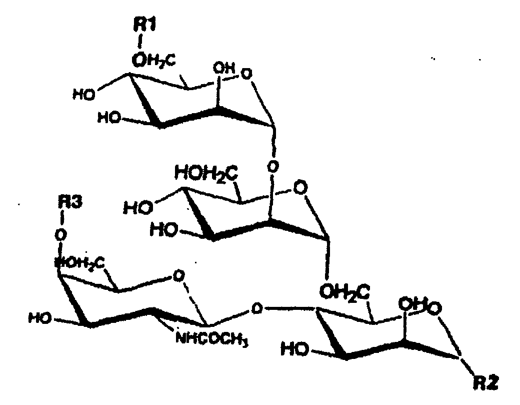 dans laquelle R1 = un hydrogène, -PO₃-CH₂-CH₂-NH₂ ou -PO₃-CH₂-CH₂-NH-X, où X = un antigène spécifique de *T*. *gondii*;
dans laquelle R2 = α-O-R4, α(l-4)-O-anhydromannitol ou α(1-4)-O-glucosamine, éventuellement lié sur α(1-6) au groupement inositol d'un ancrage membraneux phosphatidylinositol;
dans laquelle R3 = un groupement monosaccharide,
dans laquelle R4 = un hydrogène ou -L-R5, où L = un radical organique bivalent et R5 = un groupe fonctionnel permettant le couplage à un support.

2. Glycoconjugué selon la revendication 1, **caractérisé en ce que** ledit groupement monosaccharide est un groupement hexose.

3. Glycoconjugué selon 1a revendication 2, **caractérisé en ce que** ledit groupement hexose est un groupement glucose.

4. Glycoconjugué selon la revendication 3, **caractérisé en ce que** ledit groupement glucose est fixé au groupement GalNAc par une liaison α-1,4-glycosidique.

5. Glycoconjugué selon l'une quelconque des revendications 1-4, **caractérisé en ce que** ledit glycoconjugué comprend la structure suivante: 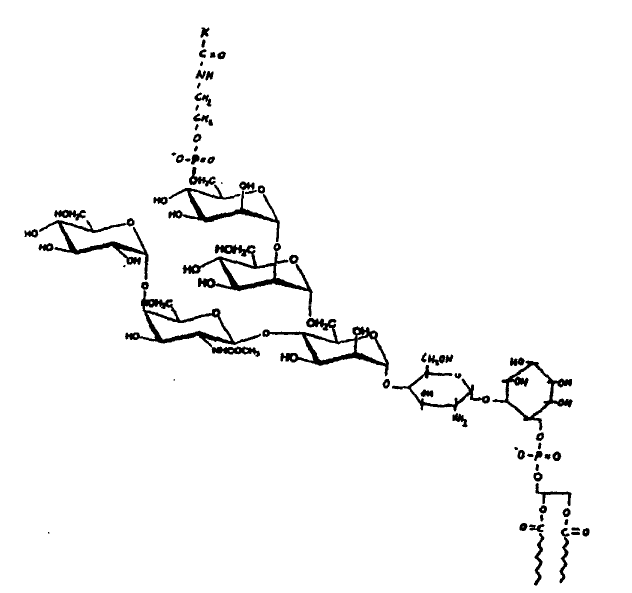 dans laquelle X = un antigène spécifique de *T. gondii*.

6. Glycoconjugué selon l'une quelconque des revendications 1-5, **caractérisé en ce que** ledit glycoconjugué peut être obtenu à partir de tachyzoïtes de *T*. *gondii* par un procédé d'isolement comprenant les étapes consistant à :
- rompre la membrane des tachyzoïtes, et
- extraire la suspension de tachyzoïtes ainsi obtenue avec un mélange chloroforme/méthanol/eau.

7. Glycoconjugué selon l'une quelconque des revendications 1-6, **caractérisé en ce que** ledit glycoconjugué est immunoréactif avec des anticorps monoclonaux humains produits par la lignée cellulaire immortalisée déposée auprès de la European Collection of Animal Cell Cultures, Porton Down (Royaume-Uni), sous le numéro de dépôt 95090608 ou 95090609.

8. Anticorps dirigé contre un glycoconjugué de *T*. *gondii*, **caractérisé en ce que** ledit anticorps est immunoréactif avec le glycoconjugué selon l'une quelconque des revendications 1-7.

9. Anticorps selon la revendication 8, **caractérisé en ce que** ledit anticorps est un anticorps monoclonal.

10. Anticorps monoclonal qui se lie à un épitope d'un glycoconjugué de *T*. *gondii*, cet épitope étant reconnu par l'anticorps monoclonal TOX.HuOT-1 ou TOX.HuOT-2 produit par les lignées cellulaires d'hybridome déposées auprès de la European Collection of Animal Cell Cultures, Porton Down (Royaume-Uni), respectivement sous les numéros de dépôt 95090608 et 95090609.

11. Anticorps monoclonal TOX.HuOT-1 ou TOX.HuOT-2 produit par les lignées cellulaires d'hybridome déposées auprès de la European Collection of Animai Cell Cultures, Porton Down (Royaume-Uni), respectivement sous les numéros de dépôt 95090608 et 95090609.

12. Lignée cellulaire immortalisée capable de produire des anticorps monoclonaux selon l'une quelconque des revendications 9-11.

13. Lignée cellulaire immortalisée déposée auprès de la European Collection of Animal Cell Cultures, Porton Down (Royaume-Uni), respectivement sous les numéros de dépôt 95090608 et 95090609.

14. Réactif immunochimique, **caractérisé en ce qu'**il comprend un glycoconjugué selon l'une quelconque des revendications 1-7.

15. Réactif immunochimique, **caractérisé en ce qu'**il comprend un anticorps selon l'une quelconque des revendications 8-11.

16. Procédé de détection de *T*. *gondii* dans un fluide d'analyse, **caractérisé en ce que** ledit fluide d'analyse est mis en contact avec un anticorps selon l'une quelconque des revendications 9-11, après quoi la présence de complexes immuns est détectée.

17. Procédé de détection d'anticorps dirigés contre *T*. *gondii* dans un fluide d'analyse, **caractérisé en ce qu'**un réactif immunochimique selon la revendication 14 est amené en contact avec le fluide d'analyse et la présence de complexes immuns formés dans le fluide d'analyse est détectée.

18. Procédé de détection de *T*. *gondii* dans un fluide d'analyse, **caractérisé en ce qu'**un réactif immunochimique selon la revendication 15 est amené en contact avec le fluide d'analyse, moyennant quoi les anticorps dirigés contre *T*. *gondii* sont amenés en contact, et la présence de complexes immuns formés est détectée.

19. Trousse d'analyse pour la détection d'une infection par *T*. *gondii*, **caractérisée en ce que** ladite trousse d'analyse comprend un réactif immunochimique selon la revendication 14.

20. Trousse d'analyse pour la détection d'une infection par *T*. *gondii*, **caractérisée en ce que** ladite trousse d'analyse comprend un réactif immunochimique selon la revendication 15.
